# EUROPEAN PATENT APPLICATION

(11) **EP 4 470 364 A1**
(43) Date of publication of application: **04.12.2024**
(21) Application number: 23743565.6
(22) Date of filing: 25.01.2023
(51) Int. Cl.: A01H 6/82, A01H 5/10, A01H 1/06, C12N 15/82, C12N 15/113, C12N 9/02, C12N 9/22

(54) **TOMATOES CONTAINING HIGH LEVELS OF 7-DEHYDROCHOLESTEROL AND PREPARATION METHOD THEREFOR**

(30) Priority: 24.01.2022 KR 20220009633
(71) Applicant: GFLAS Life Sciences, Inc., Cheongju-si, Chungcheongbuk-do 28161 (KR)
(72) Inventor: CHOE, Sunghwa, Siheung-si, Gyeonggi-do 15011 (KR); CHOI, Sunmee, Seoul 07811 (KR); KIM, Jinhwa, Seoul 06362 (KR); KIM, Jeongmo, Seoul 07572 (KR); YOU, Min Kyoung, Yongin-si, Gyeonggi-do 17079 (KR); JEON, Yun-A, Daejeon 35003 (KR)
(74) Representative: HGF
(86) International application number: PCT/KR2023/001125
(87) International publication number: WO 2023/140722

(57) **Abstract**

The present invention relates to tomatoes containing high levels of a vitamin D3 precursor, prepared by using gene-editing technology, and a preparation method therefor. Specifically, it has been confirmed that tomatoes having the DWF5-1 gene deleted accumulate high levels of 7-dehydrocholesterol and that seeds can be produced therefrom. Thus, tomatoes produced in such a way may contain high levels of the vitamin D3 precursor and have a high commercial applicability.

## Description

### Technical Field

The present invention relates to a tomato containing a high concentration of 7-dehydrocholesterol, a precursor of vitamin D3, and a method for producing the same.

### Background Art

Vitamin D is an essential nutrient for sustaining life. Insufficient vitamin D has been reported to increase not only rickets, osteomalacia, osteoporosis, but also malignant tumors such as breast cancer, collorectal cancer, and prostate cancer; cardiovascular diseases including hypertension; diabetes; multiple sclerosis; psoriasis; rheumatoid arthritis; tuberculosis; and the like. Vitamin D is a vitamin that can be ingested through food, but can also be synthesized *in vivo* through adequate sunlight. Therefore, if you are not exposed to sunlight enough, you should take foods rich in vitamin D or vitamin D nutritional supplements.

In particular, since modern people spend most of the day indoors and block sunlight with clothes and hats even when doing outdoor activities, they need to be careful about vitamin D deficiency. In the case of insufficient exposure to sunlight, vitamin D must be sufficiently ingested through food, but there is a problem in that there are not many foods with a high vitamin D content. Even if foods are known to be rich in vitamin D, the amount of vitamin D contained per 100 g is: 0.29 µg/about 7 sheets (about 100 g) of sliced cheese, 2.9 µg/100 g of tuna, 1.36 µg/2 pieces (about 100 g) of eggs, 4.4 µg/about 1 cup (about 100 g) of raisins. Therefore, it is impossible to meet the daily recommended amount of vitamin D from these foods.

In addition, as a vitamin D nutritional supplement, synthetic vitamin D has been reported to have side effects and adverse potential, and natural vitamin D has the problem of being very expensive and difficult to obtain. On the other hand, Korean Patent Publication No. 10-2017-0138657 discloses that when the DWF5 (delta 5, 7-sterol deta 7 reductase, DWARF5), CPD (constitutive photomorphogenic DWAF, DWF3) and SMT1 (sterol methyltransferase 1) genes are deleted, lettuce enriched with 7-dehydrocampesterol, a precursor of vitamin D, can be produced. However, no studies have been conducted on the production of tomatoes enriched with 7-dehydrocholesterol as a precursor of vitamin D3.

### Detailed Description of Invention

### Technical Problem

Accordingly, the present inventors conducted experiments by deleting the DWF5-1, CPD and SMT1 genes through CRISPR technology. As a result, it was confirmed that tomatoes containing a high concentration of 7-dehydrocholesterol were produced when the DWF5-1 gene was deleted. Based on the above, the present inventors completed the present invention. Specifically, the present invention is to identify DWF5-1, CPD and SMT1 deletion sites optimized for developing tomatoes containing a high concentration of 7-dehydrocholesterol, a precursor for vitamin D3 production, and to develop tomatoes enriched with a precursor of vitamin D3 comprising them.

### Solution to Problem

In order to achieve the above object, in one aspect of the present invention, there is provided a transformed tomato containing a high concentration of 7-dehydrocholesterol of Formula 1 below.

In another aspect of the present invention, there is provided a vector comprising one or more than one sgRNA (single guide RNA) that complementarily binds to a nucleotide sequence of the DWF5-1 gene and a nucleotide sequence encoding a CRISPR (clustered regularly interspaced palindromic repeats) associated protein.

In another aspect of the present invention, there is provided a method for producing a transformed tomato containing a high concentration of 7-dehydrocholesterol, comprising introducing the vector into a tomato using Agrobacterium.

### Effects of Invention

When the transformed tomato produced according to the present invention was a homozygote comprising the mutation of the DWF5-1 gene, a large amount of 7-dehydrocholesterol was accumulated in a fruit and a root of the tomato. In addition, the homozygote (T2) was able to produce seeds, and thus to preserve the desired tomato traits. Therefore, the transformed tomato of the present invention has a high commercial applicability.

### Brief Description of Drawings

Fig. 1 is a diagram showing the sites of DWFS-1 targeted by DWF5-1 sgRNA.
Fig. 2 is a diagram showing the sites of CPD targeted by CPD sgRNA.
Fig. 3 is a diagram showing the sites of SMT1 targeted by SMT1 sgRNA.
Fig. 4 is a diagram showing the results obtained by confirming the gene editing efficiency of DWF5-1 sgRNA using RGEN.
Fig. 5 is a diagram showing the results obtained by confirming the CPD gene editing efficiency of CPD sgRNA using RGEN.
Fig. 6 is a diagram showing the results obtained by confirming the SMT1 gene editing efficiency of SMT1 sgRNA using RGEN.
Fig. 7 shows a schematic diagram of a Cas9-sgRNA vector loaded with DWF5-1 sgRNA.
Fig. 8 shows a schematic diagram of a Cas9-sgRNA vector loaded with DWF5-1 sgRNA and CPD sgRNA.
Fig. 9 shows a schematic diagram of a Cas9-sgRNA vector loaded with DWF5-1 sgRNA, CPD sgRNA and SMT1 sgRNA.
Fig. 10 is a flow chart showing a process for constructing a transformed tomato using Agrobacterium.
Fig. 11 shows the germination process of a tomato constructed through gene editing.
Fig. 12 is a diagram showing the results obtained by observing a tomato plant body in which the DWF5-1 gene was edited.
Fig. 13 is a diagram showing the results obtained by observing a tomato plant body in which the DWF5-1 and CPD genes were edited.
Fig. 14 is a diagram showing the results obtained by observing a tomato plant body in which the DWF5-1, CPD and SMT1 genes were edited.
Fig. 15 is a diagram showing the results obtained by confirming using a T7E1 assay whether the DWFS-1, CPD or SMT1 gene was edited.
Fig. 16 is a diagram showing the results obtained by confirming using Sanger sequencing whether the DWF5-1 gene was deleted.
Fig. 17 is a diagram showing the results obtained by confirming the DWF5-1 gene phenotype in a T1 generation transformed tomato plant body in which the DWF5-1 gene was deleted, through next generation sequencing.
Fig. 18a is a diagram showing the gene editing efficiency and the amount of the harvested seeds (top), and nucleotide sequence (bottom) of a T1 generation homozygous transformed tomato plant body in which the DWF5-1 gene was deleted.
Fig. 18b is a diagram showing the nucleotide sequence of a T1 generation homozygous transformed tomato plant body in which the DWF5-1 gene was deleted.
Fig. 19 is a flow chart showing a sample preparation process for measuring 7-dehydrocholesterol contained in a T2 generation transformed tomato plant of a homozygote (T1) in which the DWF5-1 gene was deleted.
Fig. 20 is a diagram showing measurement conditions for measuring 7-dehydrocholesterol contained in a T2 generation transformed tomato plant body of a homozygote (T1) in which the DWF5-1 gene was deleted, by LC/MS.
Fig. 21 is a diagram showing the results obtained by measuring commercially available 7-dehydrocholesterol as a standard reagent, 7-dehydrocholesterol contained in a wild type (WT) tomato plant body, and 7-dehydrocholesterol contained in a T2 generation transformed tomato plant body of a homozygote (T1) in which the DWF5-1 gene was deleted, by LC/MS.
Fig. 22 is a graph showing the results obtained by measuring 7-dehydrocholesterol contained in a fruit of a T2 generation transformed tomato plant body of a homozygote (T1) in which the DWF5-1 gene was deleted, by LC/MS.
Fig. 23 is a diagram showing the results obtained by confirming the expression of hygromycin and phosphinothricin (PPT) resistance genes in a T2 generation transformed tomato plant body of a homozygote (T1) in which the DWF5-1 gene was deleted by RT-PCR.
Fig. 24 is a diagram showing the results obtained by comparing and analyzing the nucleotide sequence of the exon 6 domain of DWF5-1 in a T2 generation transformed tomato plant bodies (D100_3-14-29 and D100_7-1-15) in which an antibiotic resistance gene is not expressed with that of a wild type (WT) tomato plant body.
Fig. 25 is a diagram showing the results obtained by comparing and analyzing the amino acid sequence of the exon 6 domain of DWF5-1 in a T2 generation transformed tomato plant body (D100_3-14-29 and D100_7-1-15) in which an antibiotic resistance gene is not expressed with that of a wild type (WT) tomato plant body.
Fig. 26 is a diagram showing the phenotype of a homozygous (T2) transformed tomato plant body (T2) in which the DWF5-1 gene was deleted.
Fig. 27 is a graph showing the results obtained by confirming the expression level of the DWF5-1 or DWF5-2 gene in roots, stems, leaves and fruits of a tomato plant body, by q-PCR. ****p<0.0001
Fig. 28 is a graph showing the results obtained by measuring 7-dehydrocholesterol contained in roots (cultured roots) of a T3 generation transformed tomato plant body of a homozygote (T1) in which the DWF5-1 gene was deleted, by GC/MS.

### Best Mode for Carrying out the Invention

In one aspect of the present invention, there is provided a transformed tomato containing a high concentration of 7-dehydrocholesterol of Formula 1 below.

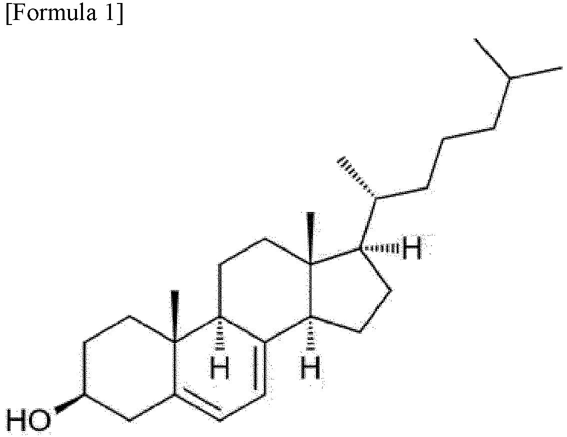

As used herein, the term "tomato" has a scientific name of *Solanum lycopersicum,* and refers to a plant belonging to the family Solanaceae, order Solanales, or a fruit thereof. It is an annual plant, has the origin of Latin America, has an height of 1 to 3 m, and has yellow flowers. The fruit is red in color due to lycopene and is used for edible purposes. In the present specification, tomato may be a plant body, and it may be plant organs (for example, roots, stems, leaves, petals (flowers), seeds, fruits, etc.), plant tissues (epidermis, sieve, soft tissue, xylem, vascular tissue), and the like. Specifically, it may be a fruit and/or a root. In this case, a tomato plant body may include cell cultures including root cultures and the like.

As used herein, the term "7-dehydrocholesterol" is a provitamin D3 that is converted to vitamin D3. In the present invention, the transformed tomato may comprise 0.01 mg to 1.5 mg of 7-dehydrocholesterol per 100 g of weight. Specifically, the transformed tomato may comprise 0.01 mg, 0.10 mg, 0.20 mg, 0.30 mg, 0.40 mg, 0.50 mg, 0.60 mg, 0.70 mg, 0.80 mg, 0.90 mg, 1.00 mg, 1.10 mg, 1.20 mg, 1.30 mg, 1.40 mg or 1.50 mg of 7-dehydrocholesterol per 100 g of weight.

In the present invention, the transformed tomato may be genetically engineered to reduce the expression or activity of the lycopersicum DWFS-1 (DWF5-1) gene or DWF5-1 protein compared to the wild type tomato. In this case, the genetic engineering may be induced by modification in the nucleic acid sequence.

As used herein, the term "DWFS" refers to 7-dehydrocholesterol reductase. This enzyme is also referred to as delta 5,7-sterol delta 7 reductase. The enzyme has an activity of reducing 5-dehydroepisterol, an intermediate metabolite of the phytosterol metabolic pathway, and converting it to 24-methylenecholesterol.

It is known that in *Arabidopsis thaliana,* where DWF5 exists as a single gene in plants, when the DWF5 gene is deleted, phytosterol metabolism rapidly decreases, resulting in dwarfism that prevents growth into adulthood. In tomato, DWF5 exists in two forms, DWFS-1 and DWF5-2. In this case, DWFS-1 and DWFS-2 may include the amino acid sequences of SEQ ID NO: 2 and SEQ ID NO: 4, respectively. It has been reported that the DWFS-1 gene will function in the phytosterol metabolic pathway and the DWFS-2 gene will function in the cholesterol production pathway by reducing 7-dehydrocholesterol (7-dehydrocholesterol reductase) and converting it to cholesterol. For this reason, when the DWFS-1 gene is deleted, it can be expected that abnormal growth may occur due to a high possibility of reducing phytosterol metabolism. However, since it has been reported that the cholesterol pathway in plants evolved from the phytosterol metabolic pathway, the possibility that DWFS-1 and DWFS-2 have functional homology with each other cannot be completely excluded. As a result of comparison and analysis of DWFS-1 and DWFS-2 gene expression patterns in the present invention, DWF5-1 was expressed more than 5-10 times higher than the DWF5-2 gene in all of leaves, stems, roots, flowers, immature fruits and mature fruits of tomato, and the DWFS-2 gene was also expressed in all tissues at a low expression level. Therefore, assuming the functional homology of DWFS-1 and DWF5-2, it was assumed that the DWFS-1 gene could more efficiently increase the 7-dehydrocholesterol content in tomato fruits.

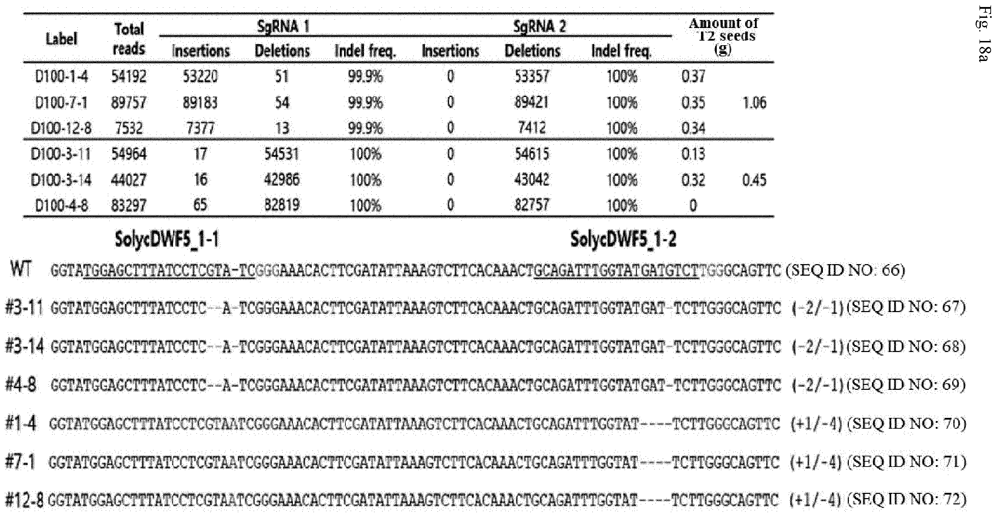

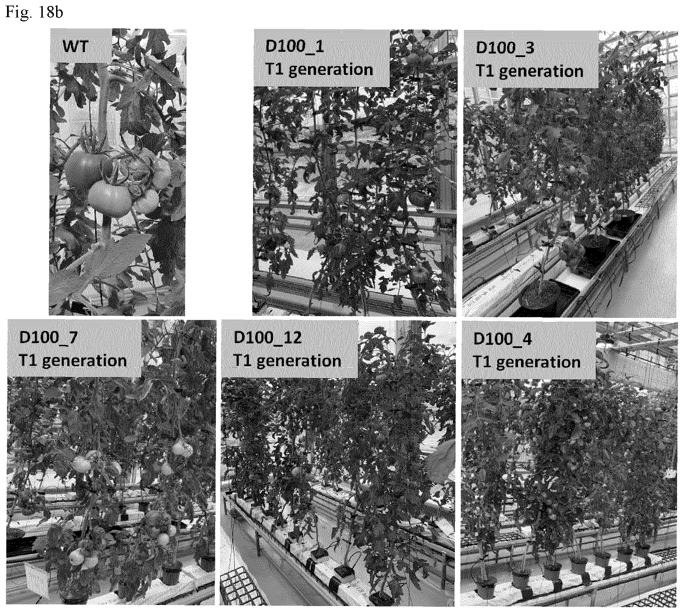

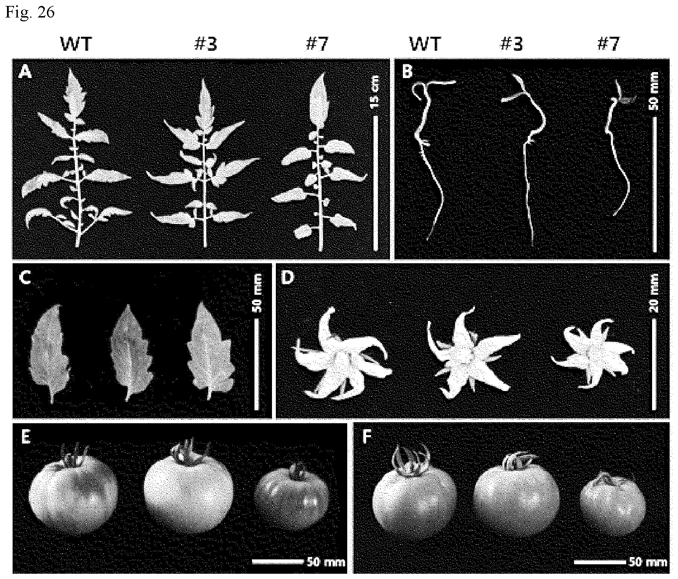

In the present invention, a mutant plant body in which only the DWFS-1 gene was deleted produced a high content of provitamin D3 in both immature and mature tomato fruits and root tissues, and their growth and seed production were similar to those of a wild type tomato (Fig. 18, Fig. 26 and Table 13). Therefore, in the present invention, a tomato in which provitamin D3 is produced in high concentration in mature fruits was developed through a deletion of at least the DWFS-1 gene, and it was confirmed that the single deletion of the DWFS-1 gene is an industrially useful technique capable of growing and producing fruits similar to those of the wild type.

In the present invention, the DWF5 may be DWF5-1. Specifically, in the present invention, the DWF5 may be DWFS-1 comprising the amino acid of SEQ ID NO: 2. The DWFS-1 gene may be genomic DNA, cDNA or RNA comprising a nucleotide sequence encoding DWF5-1. More specifically, in the present invention, the DWFS-1 gene may be genomic DNA. In the present invention, DWF5, DWFS-1 and SlDWF5-1 may be used interchangeably.

As used herein, the term "genomic DNA" refers to chromosomal DNA, and refers to a form in which genetic information is encoded in eukaryotic cells. Eukaryotic genomic DNA (hereinafter referred to as DNA) comprises exons and introns. An exon is a portion comprising a nucleic acid sequence encoding a protein, and an intron is a portion not involved in protein synthesis. In order to produce various proteins necessary for the function of organisms, DNA is transcribed into RNA, and at this time, exons excluding introns are linked together. An intron comprises information that helps transcription, such as promoters that induce initiation of transcription, and is used to make pre-mRNA in the transcription process, but is truncated rather than used to make matured mRNA.

In the present invention, the DWFS-1 gene may comprise 12 exons and 11 introns. Specifically, the DWFS-1 may comprise exon 1, exon 2, exon 3, exon 4, exon 5, exon 6, exon 7, exon 8, exon 9, exon 10, exon 11, or exon 12. In addition, the DWF5-1 may comprise intron 1, intron 2, intron 3, intron 4, intron 5, intron 6, intron 7, intron 8, intron 9, intron 10, or intron 11.

More specifically, exon 1 to exon 12 of the DWFS-1 gene may comprise or consist of the nucleic acid sequences of SEQ ID NO: 40 to SEQ ID NO: 51, respectively. Intron 1 to intron 11 of the DWFS-1 gene may comprise or consist of the nucleic acid sequences of SEQ ID NO: 52 to SEQ ID NO: 62, respectively.

In one embodiment, in the present invention, the DWFS-1 gene may comprise or consist of the nucleic acid sequence of SEQ ID NO: 63.

In the present invention, the DWFS-1 gene may comprise a flanking region that regulates transcription of DWF5-1.

As used herein, the term "flanking region" is a DNA sequence that extends on both sides of a specific gene, and is a site that is not transcribed into RNA. The DNA region adjacent to the 5' end of the gene is referred to as the 5' flanking region, and the DNA region adjacent to the 3' end of the gene is referred to as the 3' flanking region. The flanking region comprises a regulatory sequence, and regulates gene transcription by binding to a protein involved in transcription through the above sequence. In particular, in the case of eukaryotes, the 5' flanking region comprises an enhancer, a silencer, a promoter, and the like, and regulates transcription by binding to a protein such as a transcription factor and a RNA polymerase.

In one embodiment of the present invention, the 5' flanking region of the DWFS-1 gene may comprise the nucleic acid sequence of SEQ ID NO: 64. In one embodiment, the 3' flanking region of the DWFS-1 gene may comprise the nucleic acid sequence of SEQ ID NO: 65.

More specifically, in the present invention, the DWFS-1 gene may be cDNA. In one embodiment, it may comprise or consist of the nucleic acid sequence of SEQ ID NO: 1.

As used herein, the term "genetic engineering" or "genetically engineered" refers to the act of introducing one or more genetic modifications to a cell or a cell produced thereby.

As used herein, the term "reduction of the expression or activity of a gene or protein" means that the expression or activity of a target gene or protein is low compared to that of a wild type of the same species to which a target gene or protein is comparable. In addition, "inactivation" means that a protein that is not expressed or that has no activity even if expressed is produced compared to a wild-type target gene or protein.

In other words, the reduction or inactivation of the expression or activity of the DWF5-1 gene or protein means that all or part of the biological function or role normally performed by the DWF5-1 gene of the wild type tomato is lost. For example, the protein expressed by the DWFS-1 gene may be prematurely terminated or lose its normal function as a protein. Specifically, the gene editing may be to knock-out the DWFS-1 gene by generating a stop codon at the target region or generating a codon encoding an amino acid different from that of the wild type. In addition, it may be to introduce a mutation into a non-coding DNA sequence that does not produce a protein, but is not limited thereto.

Specifically, the genetically engineered transformed tomato may have the reduced DWF5-1 gene or protein by about 20% or more, about 30% or more, about 40% or more, about 50% or more, about 60% or more, about 70% or more, about 80% or more, about 85% or more, about 90% or more, about 95% or more or about 100% compared to the DWFS-1 gene or protein of the wild type tomato. The expression or activity of the DWF5-1 gene or DWF5-1 protein can be determined using any method known in the art.

The genetic engineering may be induced by modification in the nucleic acid sequence. Specifically, the genetic engineering may be induced by modification in the nucleic acid sequence through substitution (conversion), deletion or insertion. Through the above genetic engineering, the DWF5-1 gene of the transformed tomato of the present invention may comprise a gene sequence different from the DWF5-1 gene of the wild type tomato, and the biological function of the above gene may be lost.

More specifically, the engineering of the DWFS-1 gene may be induced through any one modification in nucleic acid sequence selected from the group consisting of:
i) deletion of all or 1 to 50 consecutive nucleotide sequence of the DWF5-1 gene;
ii) substitution of 1 to 50 nucleotides of the DWFS-1 gene with nucleotides different from those of the wild type DWFS-1 gene;
iii) insertion of 1 to 50 nucleotides each independently selected from A, T, C and G into the DWFS-1 gene; and
iv) a combination thereof.

More specifically, the genetic engineering may be induced through any one modification in nucleic acid sequence selected from the group consisting of:
i) deletion of all or part of the DWF5-1 gene, for example, deletion of one or more nucleotides of the DWF5-1 gene, for example, deletion of 1 to 50, 1 to 45, 1 to 40, 1 to 35, 1 to 30, 1 to 25, 1 to 20, 1 to 15, 1 to 10, 1 to 5, 1 to 3 or 1 nucleotides;
ii) substitution of one or more nucleotides, for example, 1 to 50, 1 to 45, 1 to 40, 1 to 35, 1 to 30, 1 to 25, 1 to 20, 1 to 15, 1 to 10, 1 to 5, 1 to 3 or 1 nucleotides of the DWF5-1 gene with nucleotides different from those of the wild type DWF5-1 gene;
iii) insertion of one or more nucleotides, for example, 1 to 50, 1 to 45, 1 to 40, 1 to 35, 1 to 30, 1 to 25, 1 to 20, 1 to 15, 1 to 10, 1 to 5, 1 to 3 or 1 nucleotides selected from A, T, C and G into any sites of the DWF5-1 gene; and
iv) a combination of i) to iii).

The part where the nucleic acid sequence of the DWF5-1 gene ('target region') is modified may be a region of 1 or more, 3 or more, 5 or more, 7 or more, 10 or more, 12 or more, 15 or more, 17 or more, 20 or more, 25 or more, 27 or more, 30 or more, 33 or more, 37 or more, 40 or more, 43 or more, 47 or more, or 50 or more consecutive nucleotide sequences in the above gene.

In one embodiment of the present invention, the modification in the nucleic acid sequence of the DWF5-1 gene may be included in the nucleic acid sequence of any one domain selected from the group consisting of exon 6 (SEQ ID NO: 9), exon 7 (SEQ ID NO: 10), exon 9 (SEQ ID NO: 11), exon 10 (SEQ ID NO: 12), exon 11 (SEQ ID NO: 13) and a combination thereof. In one embodiment of the present invention, the modification in the nucleic acid sequence of the DWF5-1 gene may be included in the nucleic acid sequence of the exon 6 domain. Specifically, the nucleotide sequence of SEQ ID NO: 9 comprising the nucleic acid sequence of the exon 6 domain of DWF5-1 may be modified in the nucleic acid sequence by substitution, deletion or insertion. More specifically, a modification in the sequence may be induced through substitution, deletion or insertion in the nucleotide sequence of SEQ ID NO: 9 comprising the nucleic acid sequence of the exon 6 domain of the DWF5-1 gene to lower the expression or activity of the DWF5-1 gene or DWF5-1 protein or to result in a presence of a stop codon.

The transformed tomato of the present invention may be further genetically engineered to reduce the expression or activity of the CPD gene or CPD protein. The genetic engineering is the same as described above.

As used herein, the term "CPD (constitutive photomophogenic DWAF)" is an enzyme of the CP90A family of cytochrome P450 monooxygenases, also known as DWF3. During brassinolide biosynthesis process, it acts on the C6 oxidation pathway to convert cathasterone to teasterone and convert 6-deoxocathasterone to 6-deoxoteasterone. In the present invention, the above enzyme serves to reduce 7-dehydrocampesterol to 7-dehydrocampestanol.

In this case, the CPD may comprise the amino acid sequence of SEQ ID NO: 6. In addition, the nucleic acid encoding the same may comprise the nucleotide sequence of SEQ ID NO: 5.

In the present invention, the reduction or inactivation of the expression or activity of the CPD gene or CPD protein means that all or part of the biological function or role normally performed by the CPD gene of the wild type tomato is lost. In this case, the reduction and inactivation of the expression or activity of the gene or protein are the same as described above. For example, the protein expressed by the CPD gene may be prematurely terminated or lose its normal function as a protein. Specifically, the gene editing may be to knock-out the CPD gene by generating a stop codon at the target region or generating a codon encoding an amino acid different from that of the wild type. In addition, it may be to introduce a mutation into a non-coding DNA sequence that does not produce a protein, but is not limited thereto.

Specifically, the genetically engineered transformed tomato may have the reduced CPD gene or protein by about 20% or more, about 30% or more, about 40% or more, about 50% or more, about 60% or more, about 70% or more, about 80% or more, about 85% or more, about 90% or more, about 95% or more, or about 100% compared to the CPD gene or protein of the wild type tomato. The expression or activity of the CPD gene or CPD protein can be determined using any method known in the art.

The genetic engineering may be induced by modification in the nucleic acid sequence. Specifically, the genetic engineering may be induced by modification in the nucleic acid sequence through substitution, deletion or insertion. Through the above genetic engineering, the CPD gene of the transformed tomato of the present invention may comprise a gene sequence different from the CPD gene of the wild type tomato, and the biological function of the above gene may be lost.

More specifically, the engineering of the CPD gene may be induced through any one modification in nucleic acid sequence selected from the group consisting of:
i) deletion of all or 1 to 50 consecutive nucleotide sequence of the CPD gene;
ii) substitution of 1 to 50 nucleotides of the CPD gene with nucleotides different from those of the wild type CPD gene;
iii) insertion of 1 to 50 nucleotides each independently selected from A, T, C and G into the CPD gene; and
iv) a combination thereof.

More specifically, the genetic engineering may be induced through any one modification in nucleic acid sequence selected from the group consisting of:
i) deletion of all or part of the CPD gene, for example, deletion of one or more nucleotides of the CPD gene, for example, deletion of 1 to 50, 1 to 45, 1 to 40, 1 to 35, 1 to 30, 1 to 25, 1 to 20, 1 to 15, 1 to 10, 1 to 5, 1 to 3 or 1 nucleotides;
ii) substitution of one or more nucleotides, for example, 1 to 50, 1 to 45, 1 to 40, 1 to 35, 1 to 30, 1 to 25, 1 to 20, 1 to 15, 1 to 10, 1 to 5, 1 to 3 or 1 nucleotides of the CPD gene with nucleotides different from those of the wild type CPD gene;
iii) insertion of one or more nucleotides, for example, 1 to 50, 1 to 45, 1 to 40, 1 to 35, 1 to 30, 1 to 25, 1 to 20, 1 to 15, 1 to 10, 1 to 5, 1 to 3 or 1 nucleotide(s) selected from A, T, C and G into any sites of the CPD gene; and
iv) a combination of i) to iii).

The part where the nucleic acid sequence of the CDP gene ('target region') is modified may be a region of 1 or more, 3 or more, 5 or more, 7 or more, 10 or more, 12 or more, 15 or more, 17 or more, 20 or more, 25 or more, 27 or more, 30 or more, 33 or more, 37 or more, 40 or more, 43 or more, 47 or more, or 50 or more consecutive nucleotide sequences in the above gene.

In one embodiment of the present invention, the modification in the nucleic acid sequence of the CPD gene may be included in the nucleic acid sequence of any one domain selected from the group consisting of exon 7 (SEQ ID NO: 14), exon 8 (SEQ ID NO: 15) and a combination thereof. In one embodiment of the present invention, the modification in the nucleic acid sequence of the CPD gene may be a modification in the nucleic acid sequence of the exon 8 domain. Specifically, the nucleotide sequence of SEQ ID NO: 15 comprising the nucleic acid sequence of the exon 8 domain of the CPD gene may be modified in the nucleic acid sequence by substitution, deletion or insertion. More specifically, a modification in the sequence may be induced through substitution, deletion or insertion in the nucleotide sequence of SEQ ID NO: 15 comprising the nucleic acid sequence of the exon 8 domain of the CDP gene to lower the activity of CDP or to result in a presence of a stop codon.

The transformed tomato comprising a mutation in the DWF5-1 and CPD genes of the present invention may be further genetically engineered to reduce the expression or activity of the SMT1 gene or SMT1 protein. The genetic engineering is the same as described above.

As used herein, the term "SMT1 (sterol methyltransferase 1)" is an enzyme that adds a methyl group to a side branch of a steroid. Specifically, the SMT1 serves to convert 5-dehydroepisterol to 7-dehydrocampesterol.

In this case, the SMT1 may comprise the amino acid sequence of SEQ ID NO: 8. In addition, the nucleic acid encoding the same may comprise the nucleotide sequence of SEQ ID NO: 7.

In the present invention, the reduction or inactivation of the expression or activity of the SMT1 gene or SMT1 protein means that all or part of the biological function or role normally performed by the SMT1 gene of the wild type tomato is lost. In this case, the reduction and inactivation of the expression or activity of the gene or protein are the same as described above. For example, the protein expressed by the SMT1 gene may be prematurely terminated or lose its normal function as a protein. Specifically, the gene editing may be to knock-out the SMT1 gene by generating a stop codon at the target region or generating a codon encoding an amino acid different from that of the wild type. In addition, it may be to introduce a mutation into a non-coding DNA sequence that does not produce a protein, but is not limited thereto.

Specifically, the genetically engineered transformed tomato may have the reduced SMT1 gene or SMT1 protein by about 20% or more, about 30% or more, about 40% or more, about 50% or more, about 60% or more, about 70% or more, about 80% or more, about 85% or more, about 90% or more, about 95% or more, or about 100% compared to the SMT1 gene or SMT1 protein of the wild type tomato. The expression or activity of the SMT1 gene or SMT1 protein can be determined using any method known in the art.

The genetic engineering may be induced by modification in the nucleic acid sequence. Specifically, the genetic engineering may be induced by modification in the nucleic acid sequence through substitution, deletion or insertion. Through the above genetic engineering, the SMT1 gene of the transformed tomato of the present invention may comprise a gene sequence different from the SMT1 gene of the wild type tomato, and the biological function of the above gene may be lost.

More specifically, the engineering of the SMT1 gene may be induced through any one modification in nucleic acid sequence selected from the group consisting of:
i) deletion of all or 1 to 50 consecutive nucleotide sequence region of the SMT1 gene;
ii) substitution of 1 to 50 nucleotides of the SMT1 gene with nucleotides different from those of the wild type SMT1 gene;
iii) insertion of 1 to 50 nucleotides each independently selected from A, T, C and G into the SMT1 gene; and
iv) a combination thereof.

More specifically, the genetic engineering may be induced through any one modification in nucleic acid sequence selected from the group consisting of:
i) deletion of all or part of the SMT1 gene, for example, deletion of one or more nucleotides of the SMT1 gene, for example, deletion of 1 to 50, 1 to 45, 1 to 40, 1 to 35, 1 to 30, 1 to 25, 1 to 20, 1 to 15, 1 to 10, 1 to 5, 1 to 3 or 1 nucleotides;
ii) substitution of one or more nucleotides, for example, 1 to 50, 1 to 45, 1 to 40, 1 to 35, 1 to 30, 1 to 25, 1 to 20, 1 to 15, 1 to 10, 1 to 5, 1 to 3 or 1 nucleotides of the SMT1 gene with nucleotides different from those of the wild type SMT1 gene;
iii) insertion of one or more nucleotides, for example, 1 to 50, 1 to 45, 1 to 40, 1 to 35, 1 to 30, 1 to 25, 1 to 20, 1 to 15, 1 to 10, 1 to 5, 1 to 3 or 1 nucleotides selected from A, T, C and G into any sites of the SMT1 gene; and
iv) a combination of i) to iii).

The part where the nucleic acid sequence of the SMT1 gene ('target region') is modified may be a region of 1 or more, 3 or more, 5 or more, 7 or more, 10 or more, 12 or more, 15 or more, 17 or more, 20 or more, 25 or more, 27 or more, 30 or more, 33 or more, 37 or more, 40 or more, 43 or more, 47 or more, or 50 or more consecutive nucleotide sequences in the above gene.

In one embodiment of the present invention, the modification in the nucleic acid sequence of the SMT1 gene may be included in the nucleic acid sequence of any one domain selected from the group consisting of exon 7 (SEQ ID NO: 16), exon 9 (SEQ ID NO: 17), exon 11 (SEQ ID NO: 18), exon 12 (SEQ ID NO: 19) and a combination thereof. In one embodiment of the present invention, the mutation in the SMT1 gene may include a modification in the nucleic acid sequences of the exon 7 and exon 9 domains. Specifically, the mutation in the SMT1 gene may be a modification in the nucleotide sequences of SEQ ID NO: 16 and SEQ ID NO: 17 comprising the nucleic acid sequences of the exon 7 and exon 9 domains by substitution, deletion or insertion. More specifically, a modification in the sequence may be induced through substitution, deletion or insertion in the nucleotide sequences of SEQ ID NO: 16 and SEQ ID NO: 17 comprising the nucleic acid sequences of the exon 7 and exon 9 domains of the SMT1 gene to lower the activity of SMT1 or to result in a presence of a stop codon.

The transformed tomato may be a homozygote. That is, the modification in the nucleic acid sequence may be included in two genes encoding DWF5-1. In one embodiment, the modification in the nucleic acid sequence may be included in two genes encoding any one domain selected from the group consisting of exon 6, exon 7, exon 9, exon 10, exon 11 and a combination thereof of the DWFS-1 gene. In one embodiment of the present invention, the transformed tomato may have modified sequences in two genes encoding the exon 6 domain of the DWF5-1 gene that are different from the nucleic acid sequence of the wild type DWF5-1 gene.

In the transformed tomato, the modification in the nucleic acid sequence may be included in two genes encoding DWF5-1, CPD and SMT1. In this case, the transformed tomato may have modified sequences in two genes encoding any one domain selected from the group consisting of exon 7, exon 8 and a combination thereof of the CPD gene that are different from the nucleic acid sequence of the wild type CPD gene. In addition, the modification in the nucleic acid sequence may be included in two genes encoding any one domain selected from the group consisting of exon 7, exon 9, exon 11, exon 12 and a combination thereof of the SMT1 gene. In this case, the modification in the nucleic acid sequence is the same as described above.

In one embodiment of the present invention, in the transformed tomato, the modification in the nucleic acid sequence may be included in two genes each encoding the exon 6 domain of DWF5-1, the exon 7 domain of CDP, and the exon 7 and exon 9 domains of SMT1.

When the transformed tomato according to the present invention is a homozygote and the expression or activity of the DWFS-1 gene or protein is reduced or inactivated, the tomato can form seeds. When the transformed tomato is a homozygote and the expression or activity of the DWF5-1, CPD and SMT1 genes or proteins is reduced or inactivated, the tomato may not bear fruit and may not form seeds.

In another aspect of the present invention, there is provided a vector comprising one or more than one sgRNA that complementarily binds to a nucleotide sequence of the DWFS-1 gene and a nucleotide sequence encoding a CRISPR-associated protein. Here, DWFS-1 is the same as described above. In addition, the DWFS-1 gene may be genomic DNA, cDNA or RNA.

As used herein, the term "guide RNA (gRNA)" refers to a polynucleotide that recognizes a target nucleic acid in a cell through genome editing and cleaves, inserts, or ligates the target nucleic acid. The gRNA may comprise a sequence complementary to a target sequence in a target nucleic acid. The gRNA may be a polynucleotide complementary to a nucleotide sequence of 2 to 24 consecutive nucleotides (hereinafter referred to as 'nt') in the 5' or 3' direction of the PAM in the target nucleic acid. The length of the gRNA may be 17 nt, 18 nt, 19 nt, 20 nt, 21 nt, 22 nt, 23 nt or 24 nt.

The gRNA may be a single guide RNA (sgRNA). The sgRNA may include a CRISPR RNA (crRNA) specific to a target nucleic acid sequence and/or a transactivating crRNA (tracrRNA) that forms a complex with a CRISPR-associated protein. The sgRNA may comprise a portion having a nucleotide sequence (targeting sequence) complementary to a target nucleic acid sequence in a target gene (target region) (also referred to as a spacer region, a target DNA recognition sequence, a base pairing region, etc.) and a hairpin structure for binding to a CRISPR-associated protein. More specifically, it may comprise a portion comprising a nucleotide sequence (targeting sequence) complementary to a target nucleic acid sequence in a target gene, a hairpin structure for binding to a CRISPR-associated protein, and a terminator sequence. The structure described above may be sequentially present in order from 5' to 3', but is not limited thereto. Any type of sgRNA may be used as long as the sgRNA comprises the main regions of crRNA and tracrRNA and a nucleotide sequence complementary to the target gene.

In the present invention, the sgRNA may complementarily bind to the nucleic acid sequence of the genomic DNA of DWF5-1. Specifically, the sgRNA may complementarily bind to SEQ ID NO: 63. More specifically, the sgRNA may complementarily bind to a nucleic acid sequence of one or more than one domain selected from the group consisting of exon 1 to exon 12, intron 1 to intron 11, 5' flank region and 3' flank region of the DWF5-1 gene. More specifically, it may complementarily bind to a nucleic acid sequence of one or more than one domain selected from the group consisting of SEQ ID NO: 40 to SEQ ID NO: 62, SEQ ID NO: 64 and SEQ ID NO: 65.

In the present invention, the sgRNA may complementarily bind to the nucleic acid sequence of cDNA of DWF5-1. Specifically, the sgRNA may complementarily bind to the nucleic acid sequence of SEQ ID NO: 1.

In one embodiment of the present invention, the sgRNA may complementarily bind to a nucleic acid sequence of any one domain selected from the group consisting of exon 6, exon 7, exon 9, exon 10 and exon 11 of the DWF5-1 gene. In one embodiment, the sgRNA binding to the nucleic acid sequence of the exon 6 domain of the DWF5-1 gene may comprise the nucleotide sequence of SEQ ID NO: 20 or SEQ ID NO: 21. In one embodiment, the sgRNA binding to the nucleic acid sequence of the exon 7 domain may comprise the nucleotide sequence of SEQ ID NO: 22. In one embodiment, the sgRNA binding to the nucleic acid sequence of the exon 9 domain may comprise the nucleotide sequence of SEQ ID NO: 23 or SEQ ID NO: 24. In one embodiment, the sgRNA binding to the exon 10 domain may comprise the nucleotide sequence of SEQ ID NO: 25. In one embodiment, the sgRNA binding to the nucleic acid sequence of the exon 11 domain may comprise the nucleotide sequence of SEQ ID NO: 26. Preferably, in one embodiment of the present invention, the sgRNA may comprise or consist of the nucleotide sequences of SEQ ID NO: 20 and SEQ ID NO: 21 that complementarily binds to the nucleic acid sequence of the exon 6 domain.

In the present invention, the vector may further comprise sgRNA that complementarily binds to the nucleotide sequence of the CPD gene. The sgRNA that complementarily binds to the CPD gene may complementarily bind to the nucleotide sequence of the exon 7 or exon 8 domain of the CPD gene. The sgRNA binding to the exon 7 domain of the CPD gene may comprise the nucleotide sequence of SEQ ID NO: 27. The sgRNA binding to the exon 8 domain of the CPD gene may comprise the nucleotide sequence of SEQ ID NO: 28, SEQ ID NO: 29, SEQ ID NO: 30 or SEQ ID NO: 31. In one embodiment of the present invention, the sgRNA may comprise the nucleotide sequences of SEQ ID NO: 28 and SEQ ID NO: 29 that complementarily binds to the nucleotide sequence of the exon 8 domain.

In the present invention, the vector comprising the sgRNA that complementarily binds to the nucleotide sequences of the DWF5 and CPD genes may further comprise the sgRNA that complementarily binds to the nucleotide sequence of the SMT1 gene. In this case, the sgRNA that complementarily binds to the nucleotide sequence of the SMT1 gene may complementarily bind to a nucleotide sequence of one or more than one domain selected from the group consisting of exon 7, exon 9, exon 11 and exon 12 of the SMT1 gene. The sgRNA binding to the exon 7 domain of the SMT1 gene may comprise the nucleotide sequence of SEQ ID NO: 32. The sgRNA binding to the exon 9 domain of the SMT1 gene may comprise the nucleotide sequence of SEQ ID NO: 33. The sgRNA binding to the exon 11 domain of the SMT1 gene may comprise the nucleotide sequence of SEQ ID NO: 34. The sgRNA binding to the exon 12 domain of the SMT1 gene may comprise the nucleotide sequence of SEQ ID NO: 35. In one embodiment of the present invention, the sgRNA may comprise the nucleotide sequences of SEQ ID NO: 32 and SEQ ID NO: 33 that complementarily binds to the nucleotide sequences of the exon 7 and exon 9 domains. In this case, the sgRNA that complementarily binds to the nucleotide sequences of the DWF5 and CPD genes is the same as described above.

As used herein, the term "CRISPR (clustered regularly interspaced palindromic repeats) associated protein" (CRISPR-associated protein) refers to an enzyme that can recognize and cleave a nucleic acid when a nucleic acid such as DNA or RNA has a double strand or a single strand (dsDNA/RNA and ssDNA/RNA). Specifically, it can recognize double-stranded or single-stranded nucleic acid bound to sgRNA and cleave it. The CRISPR-associated protein includes a CRISPR-associated protein and a mutant having its function.

The CRISPR-associated protein may be derived from bacteria of *Streptococcus pyogenes, Streptococcus thermophilus, Streptococcus sp., Staphylococcus aureus, Nocardiopsis dassonvillei, Streptomyces pristinae spiralis, Streptomyces viridochromogenes, Streptosporangium roseum, Alicyclobacillus acidocaldarius, Bacillus pseudomycoides, Bacillus selenitireducens, Exiguobacterium sibiricum, Lactobacillus delbrueckii, Lactobacillus salivarius, Microscilla marina, Burkholderiales bacterium, Polaromonas naphthalenivorans, Polaromonas sp., Crocosphaera watsonii, Cyanothece sp., Microcystis aeruginosa, Pseudomonas aeruginosa, Synechococcus sp., Acetohalobium arabaticum, Ammonifex degensii, Caldicelulosiruptor becscii, Candidatus Desulforudis, Clostridium botulinum, Clostridium difficile, Finegoldia magna, Natranaerobius thermophilus, Pelotomaculum thermopropionicum, Acidithiobacillus caldus, Acidithiobacillus ferrooxidans, Allochromatium vinosum, Marinobacter sp., Nitrosococcus halophilus, Nitrosococcus watsoni, Pseudoalteromonas haloplanktis, Ktedonobacter racemifer, Methanohalobium evestigatum, Anabaena variabilis, Nodularia spumigena, Nostoc sp., Arthrospira maxima, Arthrospira platensis, Arthrospira sp., Lyngbya sp., Microcoleus chthonoplastes, Oscillatoria sp., Petrotoga mobilis, Thermosipho africanus, Acaryochloris marina, Leptotrichia shahii, Prevotella* or *Francisella novicida.*

In the present invention, the CRISPR-associated protein may be any one selected from the group consisting of Cas9, Cpf1, c2c1, C2c2, Cas13, c2c3, Cas1, Cas1B, Cas2, Cas3, Cas4, Cas5, CasSe (CasD), Cas6, Cas6e, Cas6f, Cas7, Cas8a, Cas8a1, Cas8a2, Cas8b, Cas8c, Csn1, Csx12, Cas10, Cas10d, Cas10, Cas10d, CasF, CasG, CasH, Csyl, Csy2, Csy3, Cse1 (CasA), Cse2 (CasB), Cse3 (CasE), Cse4 (CasC), Csc1, Csc2, Csa5, Csn2, Csm2, Csm3, Csm4, Csm5, Csm6, Cmrl, Cmr3, Cmr4, Cmr5, Cmr6, Csb1, Csb2, Csb3, Csx17, Csx14, Csx10, Csx16, CsaX, Csx3, Csxl, Csx15, Csf1, Csf2, Csf3, Csf4 and Cul966. Specifically, the CRISPR-associated protein may be Cas9.

The Cas9 protein may form a complex with the guide sgRNA to act as a form of a ribonucleic acid protein (RNP). Here, "ribonucleoprotein (RNP)" is a complex of RNA and a protein, and may be a Cas9-sgRNA complex in the present invention.

As used herein, the term "vector" is capable of being introduced into a host cell and then recombined and inserted into a genome of the host cell. Alternatively, the vector is understood to be a nucleic acid vehicle comprising a polynucleotide sequence capable of autonomous replication as an episome. Such vectors include linear nucleic acids, plasmids, phagemids, cosmids, RNA vectors, viral vectors, mini-chromosomes and analogues thereof. Examples of viral vectors include, but are not limited to, retroviruses, adenoviruses, and adeno-associated viruses.

In one embodiment of the present invention, the vector may comprise any one of the nucleotide sequences of SEQ ID NO: 36 to SEQ ID NO: 38. In addition, in the present invention, the vector has about 70%, about 75%, about 80%, about 85%, about 90%, about 91%, about 92%, about 93%, about 94%, about 95%, about 96%, about 97%, about 98%, about 99% or about 100% sequence homology to any one of SEQ ID NO: 36 to SEQ ID NO: 38. In addition, the vector may be partially or completely codon-optimized for expression in a target organism or cell.

The vector may be operably linked to an appropriate promoter so that the polynucleotide can be expressed in a host cell. When the vector of the present invention is applied to a plant cell, the promoter of the present invention may include a promoter used for gene introduction into a plant body. Examples of promoters may include, but are not limited to, SP6 promoter, T7 promoter, T3 promoter, PM promoter, maize ubiquitin promoter (Ubi), cauliflower mosaic virus (CaMV) 35S promoter, nopaline synthase (nos) promoter, figwort mosaic virus 35S promoter, sugarcane bacilliform virus promoter, commelina yellow mottle virus promoter, ribulose-1,5-bisphosphate carboxylase small subunit (ssRUBISCO) photoinducible promoter, rice cytosolic triosephosphate isomerase (TPI) promoter, Arabidopsis adenine phosphoribosyltransferase (APRT) promoter, and octopine synthase promoter.

Specifically, the vector may be plasmid DNA, phage DNA, and the like. In addition, the vector may be commercially developed plasmids (pUC18, pBAD, pIDTSAMRT-AMP, etc.), *Escherichia* coli-derived plasmids (pYG601BR322, pBR325, pUC118, pUC119, etc.), *Bacillus subtilis*-derived plasmids (pUB 110, pTP5, etc.), yeast-derived plasmids (YEp13, YEp24, YCp50, etc.), phage DNA (Charon4A, Charon21A, EMBL3, EMBL4, λgt10, λgt11, λZAP, etc.), animal viral vectors (retrovirus, adenovirus, vaccinia virus, etc.), insect viral vectors (baculovirus, etc.), and the like. Since the expression level and modification of the protein of the vector appear differently depending on the host cell, it is preferable to select and use the host cell most suitable for the purpose.

In the present invention, the vector may include a selectable marker. The selectable marker is a nucleic acid sequence having characteristics that can be selected by a conventional chemical method, and includes all genes capable of distinguishing transformed cells from non-transformed cells. Examples of markers may include, but are not limited to, herbicide-resistance genes such as glyphosate, glufosinate ammonium or phosphinothricin, and antibiotic-resistance genes such as kanamycin, G418, bleomycin, hygromycin, and chloramphenicol. In one embodiment of the present invention, the selectable marker may be hygromycin and/or phosphinothricin.

In another aspect of the present invention, there is provided a method for producing a transformed tomato containing a high concentration of 7-dehydrocholesterol, comprising introducing a vector comprising the gene encoding the sgRNA and the gene encoding the CRISPR-associated protein into a tomato using Agrobacterium.

The transformed plant may be constructed through a plant transformation method known in the art. Those of ordinary skill in the art can select and carry out a known transformation method suitable for a particular plant in consideration of the characteristics of the plant selected as the host. Specifically, a transformation method using Agrobacterium can be used as a plant transformation method.

The "transformation method using Agrobacterium" is a method of transferring an external gene to plant cells using Agrobacterium, a gram-negative soil bacterium that causes tumors in the roots and stems of plants. This is a method using a phenomenon in which the T-DNA (transfer DNA) of a tumor-inducing plasmid (Ti plasmid) found in Agrobacterium such as *Agrobacterium tumefaciens* and *Agrobacterium rhizogenes* is inserted into the plant genome.

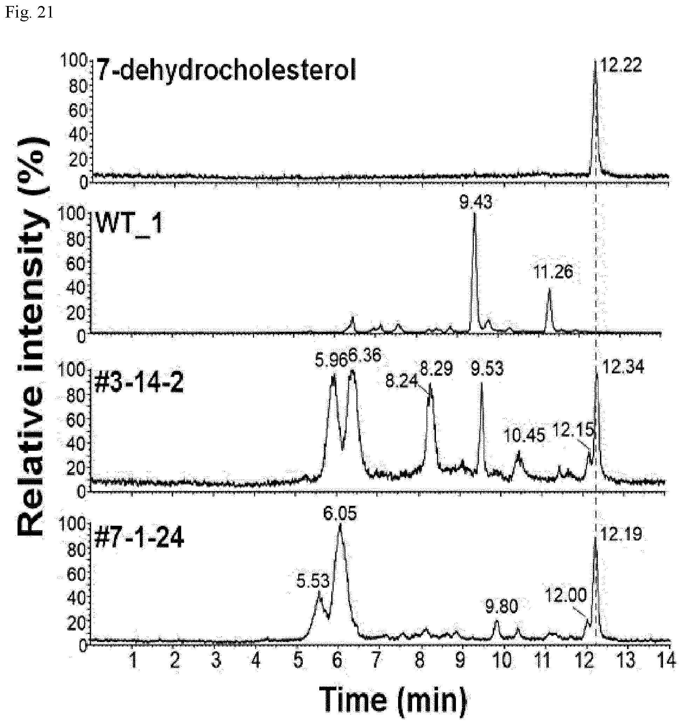

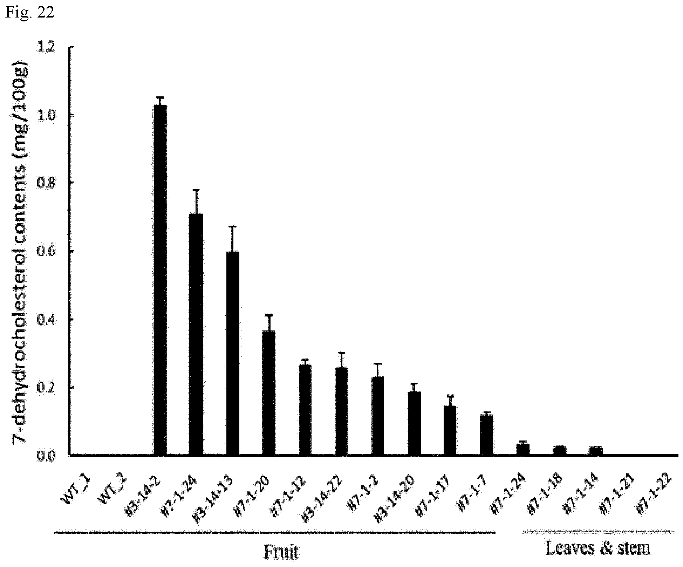

In one embodiment of the present invention, when a modification in the nucleic acid sequence is included in the exon 6 domain of the DWF5-1 gene, a homozygous tomato (T1) can produce seeds (Fig. 18). In addition, a homozygous T2 generation tomato having a modification in the nucleic acid sequence in the exon 6 domain of the DWF5-1 gene can grow normally and produce seeds (Fig. 26), and 7-dehydrocholesterol accumulates in a high concentration in fruits and roots (Table 14, Fig. 21 and Fig. 22). However, when the transformed tomato included a modification in the nucleic acid sequence in the exon 6 domain of the DWF5-1 gene and the exon 8 domain of the CPD gene, the tomato failed to grow into an intact plant body. In addition, when the transformed tomato was a homozygote having a modification in the nucleic acid sequence in the exon 6 domain of the DWF5-1 gene, the exon 8 domain of the CPD gene, and the exon 7 and exon 9 domains of the SMT1 gene, no fruit was produced.

### Mode for Carrying out the Invention

Hereinafter, the present invention will be described in more detail by way of the following examples. However, the following examples are only for illustrating the present invention, and the scope of the present invention is not limited only to these examples.

### Example 1. Construction of Cas9-sgRNA vector for gene editing of DWF5, CPD and SMT1

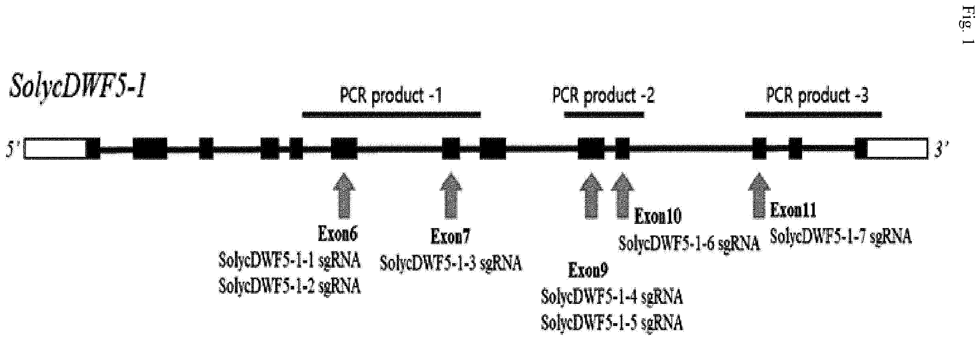

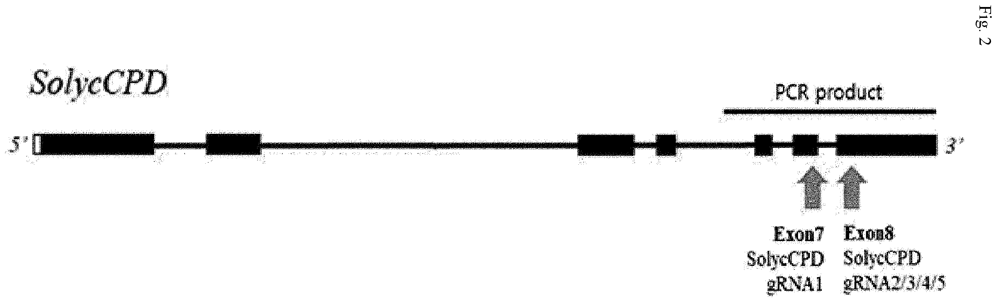

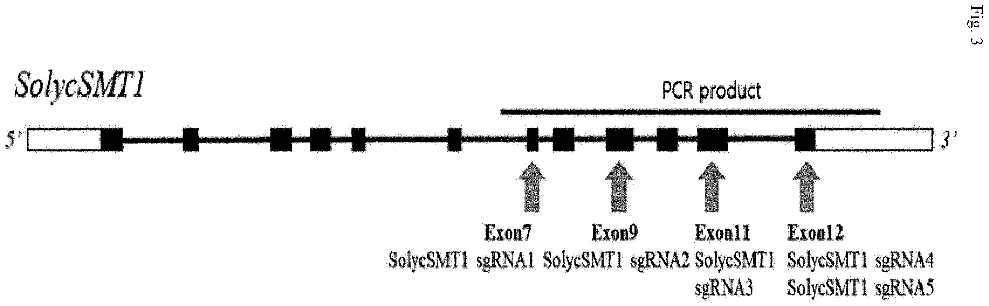

Design and selection of sgRNAs for gene editing of DWF5-1 (hereinafter referred to as 'DWF5', Fig. 1), CPD (Fig. 2) and SMT1 (Fig. 3) were conducted through the Cas-Designer website (http://www.rgenome.net/cas-dessdaigner/). The name or sequence of the target gene and the species of animal or plant were selected, and then the Cas9 to be used was selected, and sgRNA was designed based on the results of the required exon sites in the gene, GC content of sgRNA, and off-target where mismatch exists. Cas9 has a PAM sequence of NGG-3', and tomato (*S. lycopersicum*) was selected as the plant (Figs. 1 to 3). In addition, sgRNA was screened to confirm the gene editing efficiency (Table 2 and Figs. 1 to 3).

Specifically, the targeting adequacy of sgRNA was conducted using RGEN (RNA-guided endonuclease) analysis. The DNA template was purified using a PCR purification kit from the above amplified PCR product (PCR product), and then the amount of the DNA template was measured, and RGEN was performed in the combination shown in Table 1. After incubation at 37°C for 1 hour, 3 µL of 6x DNA-Purple dye was put into each, frozen, and then loaded onto a 2.5% Gel. The gene editing efficiency was analyzed by analyzing the degree of uncleaved bands in the DNA template.

**[Table 1]**

| RGEN final concentration condition | |
|---|---|
| 10X 3.1 NEB buffer | 2 µL |
| sgRNA (120 nM) | 5 µL |
| spyCas9 (100 nM) | 5 µL |
| DNA template (PCR product) | 200 ng |
| DW up to 20 µL, 37°C 1h | |

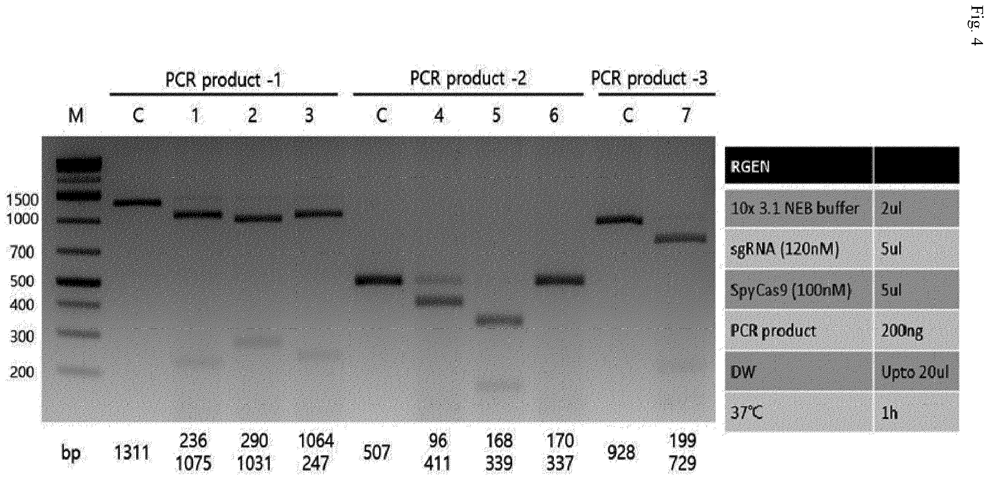

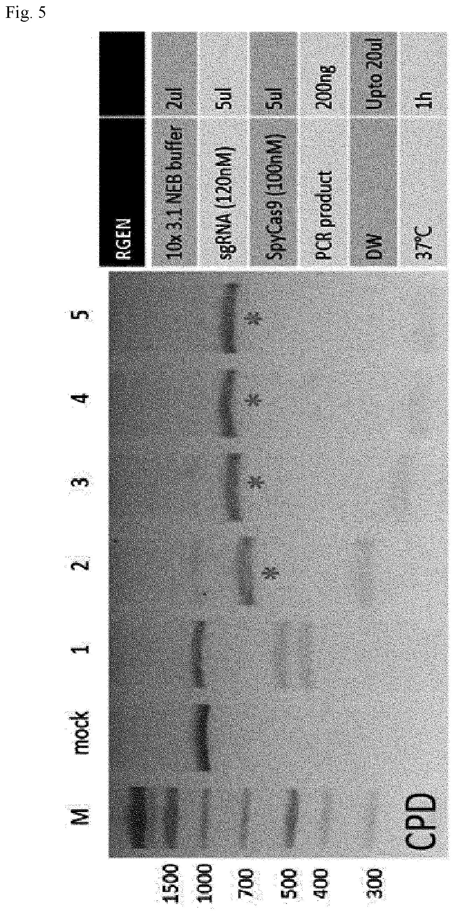

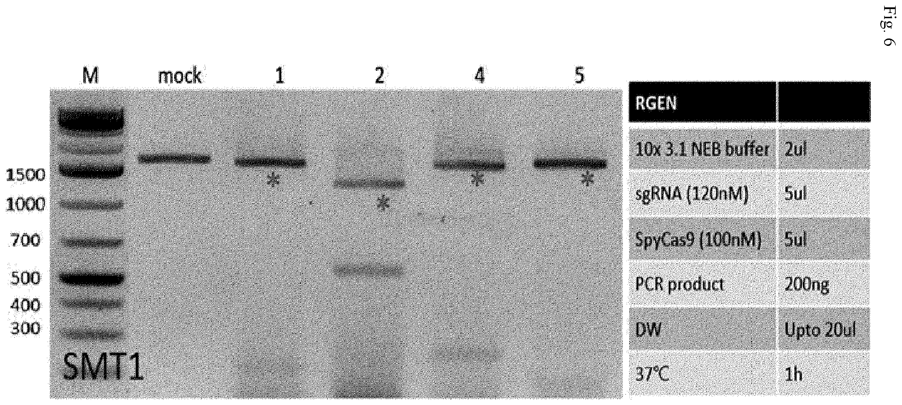

As a result, in the case of sgRNAs constructed to target the DWF5 gene, it was confirmed that all sgRNAs except for D5-4 and D5-6 could target the DWF5 gene (Fig. 4). All CPD sgRNAs except C1 targeted the CPD gene (Fig. 5), and all of the constructed SMT1 sgRNAs targeted the CPD gene (Fig. 6).

**[Table 2]**

| Target Gene (Cas9) | sgRNA (primer name) | DNA cleavage assay | sgRNA (sub name) | sgRNA sequence (PAM site 3') | sites in gene (exon) |
|---|---|---|---|---|---|
| SolycDWF5 (SEQ ID NO: 1) | SolycDWF5-1 | O | D5-1 | TGGAGCTTTATCCTCGTAT CGGG (SEQ ID NO: 20) | 6 (SEQ ID NO: 9) |
| | SolycDWF5-2 | O | D5-2 | GCAGATTTGGTATGATGT CTTGG (SEQ ID NO: 21) | 6 (SEQ ID NO: 9) |
| | SolycDWF5-3 | O | D5-3 | TGTAAATACGATATTGAT GTTGG (SEQ ID NO: 22) | 7 (SEQ ID NO: 10) |
| | SolycDWF5-4 | Δ | D5-4 | GACCAGCCACTAGTATGT AAAGG (SEQ ID NO: 23) | 9 (SEQ ID NO: 11) |
| | SolycOWF5-5 | O | D5-5 | TGCATTTTCCATTTGTTCT CCGG (SEQ ID NO: 24) | 9 (SEQ ID NO: 11) |
| | SolycDWF5-6 | X | D5-6 | CCTCTGGTGAGAAAAAGA CCAGC (SEQ ID NO: 25) | 10 (SEQ ID NO: 12) |
| | SolycOWF5-7 | O | D5-7 | AGAAAGATGACGTAGAAG TAGGG (SEQ ID NO: 26) | 11 (SEQ ID NO: 13) |
| SolycCPD (SEQ ID NO: 5) | SolycCPD gRNA1 | Δ | C1 | AACGAGTCACCAAGTGGT GAAGG (SEQ ID NO: 27) | 7 (SEQ ID NO: 14) |
| | SolycCPD gRNA2 | O | C2 | CGCTTCAGCATCCTTGTCG TTGG (SEQ ID NO: 28) | 8 (SEQ ID NO: 15) |
| | SolycCPD gRNA3 | O | C3 | TGAGCAGAAAGAAGAAG AGAAGG (SEQ ID NO: 29) | 8 (SEQ ID NO: 15) |
| | SolycCPD gRNA4 | O | C4 | TGAGATTGTAGAATGCAG AGTGG (SEQ ID NO: 30) | 8 (SEQ ID NO: 15) |
| | SolycCPD gRNA5 | O | C5 | GTAGAATGCAGAGTGGAG GGAGG (SEQ ID NO: 31) | 8 (SEQ ID NO: 15) |
| SolycSMTl (SEQ ID NO: 7) | SolycSMT1 gRNA1 | O | S1 | GTTGAACCGCAAAGTGGG ATTGG | 7 (SEQ ID NO: 16) |
| | | | | (SEQ ID NO: 32) | |
| | SolycSMT1 gRNA2 | O | S2 | GATGCTATAGAGAGATCT ATAGG (SEQ ID NO: 33) | 9 (SEQ ID NO: 17) |
| | SolycSMT1 gRNA4 | O | S4 | GTTTCGGCACTTGAATAT GTGGG (SEQ ID NO: 34) | 11 (SEQ ID NO: 18) |
| | SolycSMTl gRNA5 | O | S5 | GCCGCAGAAGGTCTTGTT GGTGG (SEQ ID NO: 35) | 12 (SEQ ID NO: 19) |

Based on the results of the sgRNA screening, a Cas9-sgRNA vector comprising sgRNAs constructed to target DWF5, CPD or SMT1 was finally constructed. The pNGPJ0014 vector (SEQ ID NO: 39) was constructed based on the pCAMBIA1300 plasmid having kanamycin- and hygromycin-resistance genes.

The pNGPJ0014 vector has an antibiotic cassette for selection by kanamycin and hygromycin antibiotics, and a polycistronic tRNA-gRNA cassette synthesized with Cas9. The Cas9 was constructed to be expressed by the Arabidopsis ubiquitin 10 (Ubi 10) promoter, and the tRNA-gRNA was constructed to be expressed by the Arabidopsis ubiquitin 6 promoter.

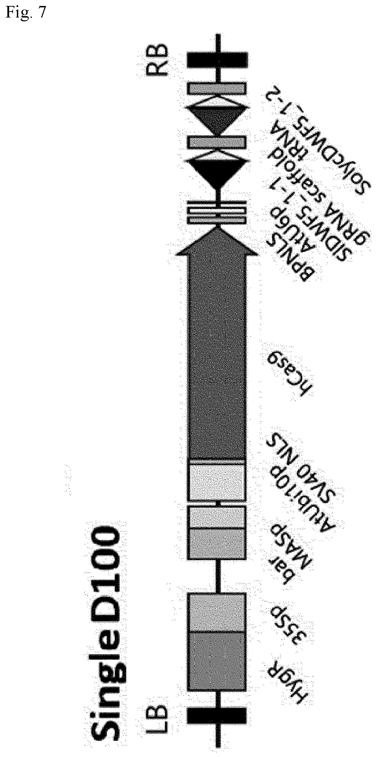

In order to deliver the Cas9 protein to the nucleus, the SV40 (PKKKRKV) nuclear localization signal sequence was added to the N-terminus of the Cas9 open reading frame, and the Bipartite (KEPAATKKAGQAKKKK) nuclear localization signal sequence was added to the C-terminus. At this time, the Cas9-sgRNA vector was constructed to target 1 to 3 types of genes. In addition, it was constructed to load two types of sgRNA per target. The Cas-sgRNA vector comprising the nucleotide sequences of D5-1 sgRNA (SEQ ID NO: 20) and D5-2 sgRNA (SEQ ID NO: 21) targeting the DWF5 gene was designated as "D100" (SEQ ID NO: 36) (Fig. 7).

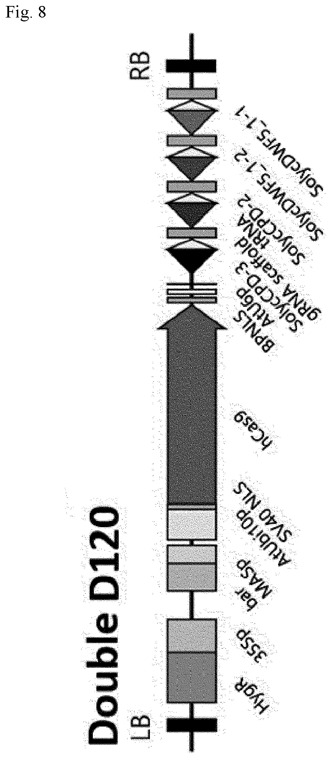

The Cas9-sgRNA vector targeting the CPD and DWF5 genes was designated as "D120" (SEQ ID NO: 37). At this time, the CPD sgRNA comprises the nucleotide sequences of C2 (SEQ ID NO: 28) and C3 (SEQ ID NO: 29), and the DWF5 sgRNA comprises the nucleotide sequences of D5-1 (SEQ ID NO: 20) and D5-2 (SEQ ID NO: 21) (Fig. 8).

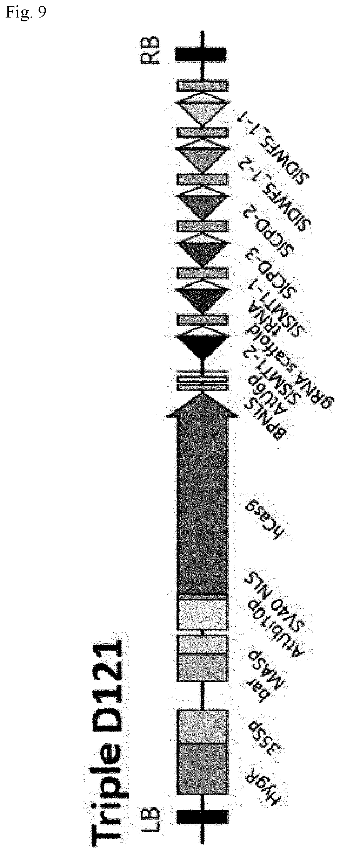

The Cas9-sgRNA vector targeting the SMT1, CPD and DWF5 genes was designated as "D121" (SEQ ID NO: 38). At this time, the SMT1 sgRNA comprises the nucleotide sequences of S1 (SEQ ID NO: 32) and S2 (SEQ ID NO: 33), the CPD sgRNA comprises the nucleotide sequences of C2 (SEQ ID NO: 28) and C3 (SEQ ID NO: 29), and the DWF5 sgRNA comprises the nucleotide sequences of D5-1 (SEQ ID NO: 20) and D5-2 (SEQ ID NO: 21) (Fig. 9).

### Example 2. Transformation of tomato through DWF5, CPD and SMT1 gene editing

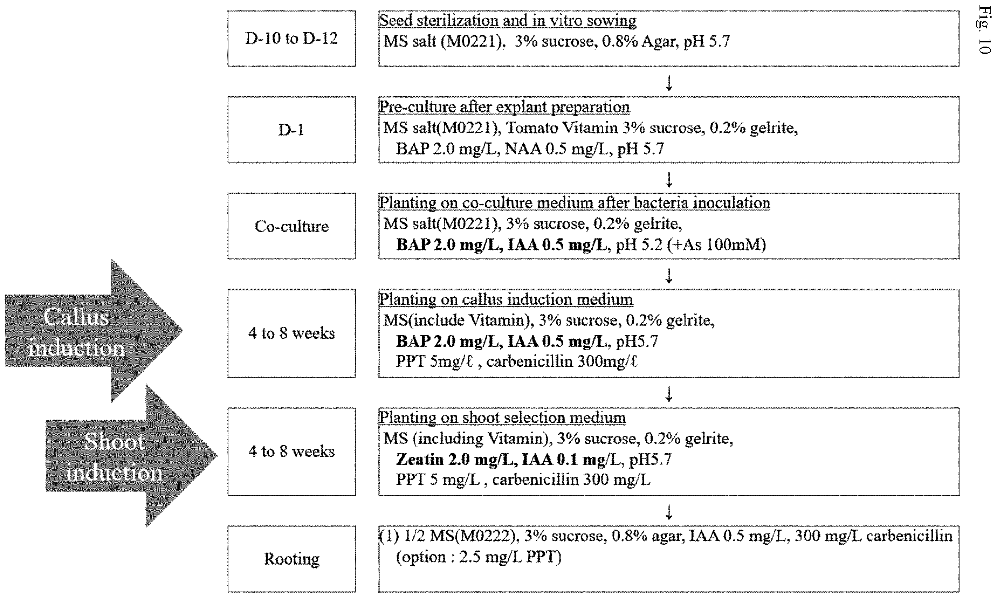

Transformation of tomato was performed as described in the schematic diagram of Fig. 10. Media and hormones used in each step were prepared and used in the same manner as described in Tables 3 to 8.

**[Table 3]**

| | | | |
|---|---|---|---|
| TMS (V) medium | MS SALT | 4.3 g/1L | |
| | Myo-inositol | 100 mg/L | Four types of vitamins were added after preparing a 100-fold stock solution. |
| | Nicotinic acid | 0.5 mg/L | |
| | Pyridoxine HCl | 0.5 mg/L | ; a 100-fold (100X) vitamin stock solution was used at 10 mL/L each. |
| | Thiamine HCl | 0.1 mg/L | |
| | Sucrose | 30 g/L | |
| | Plant Agar | 0.6 to 0.7%/L | |
| | The medium was prepared, and then the pH was adjusted to 5.7 using 1 N NaOH, and then sterilized in an autoclave at 121°C for 15 minutes, and about 30 mL was dispensed into a 90×15 mm petridish. After sterilizing the medium, the vitamin stock solution was added when the temperature of medium was between 55°C and 60°C. | | |

**[Table 4]**

| | | | |
|---|---|---|---|
| TPC medium (Tomato Pre-Culture Medium) | MS SALT | 4.3 g/lL | |
| | Myo-inositol | 100 mg/L | Four types of vitamins were added after preparing a 100-fold stock solution. |
| | Nicotinic acid | 0.5 mg/L | |
| | Pyridoxine HCl | 0.5 mg/L | ; a 100-fold (100X) vitamin stock solution was used at 10 mL/L each. |
| | Thiamine HCl | 0.1 mg/L | |
| | Sucrose | 30 g/L | |
| | Gelrite | 0.25%/L | |
| | BA | 1 mg/L | |
| | NAA | 0.1 mg/L | |
| | The medium was prepared, and then the pH was adjusted to 5.7 using 1 N NaOH, and then sterilized in an autoclave at 121°C for 15 minutes, and about 30 mL was dispensed into a 90×15 mm petridish. After sterilizing the medium, the vitamin stock solution was added when the temperature of medium was between 55°C and 60°C. | | |

**[Table 5]**

| | | | |
|---|---|---|---|
| TCC medium (Tomato Co-Culture Medium) | MS SALT | 4.3 g/lL | |
| | Myo-inositol | 100 mg/L | Four types of vitamins were added after preparing a 100-fold stock solution. |
| | Nicotinic acid | 0.5 mg/L | |
| | Pyridoxine HCl | 0.5 mg/L | ; a 100-fold (100X) vitamin stock solution was used at 10 mL/L each. |
| | Thiamine HCl | 0.1 mg/L | |
| | Sucrose | 30 g/L | |
| | Gelrite | 0.25%/L | |
| | BA | 1 mg/L | |
| | NAA | 0.1 mg/L | |
| | Acetosyringone (AS) | 100 mM/L | |
| | The medium was prepared, and then the pH was adjusted to 5.2 using 1 N NaOH, and then sterilized in an autoclave at 121°C for 15 minutes, and about 30 mL was dispensed into a 90×15 mm petridish. After sterilizing the medium, the vitamin stock solution, BA, NA and AS were added when the temperature of medium was between 55°C and 60°C. | | |

**[Table 6]**

| | | | |
|---|---|---|---|
| Bacterial dilution medium | MS SALT | 4.3 g/L | The pH was adjusted to 5.7 using 1 N NaOH and 1 N HCl, and then it was sterilized in an autoclave at 121°C for 15 minutes and stored in a refrigerator. |
| | Sucrose | 30 g/L | |

**[Table 7]**

| | | | |
|---|---|---|---|
| TSI medium (Tomato Shoot Induction Medium) | MS SALT | 4.3 g/lL | |
| | Myo-inositol | 100 mg/L | Four types of vitamins were added after preparing a 100-fold stock solution. |
| | Nicotinic acid | 0.5 mg/L | |
| | Pyridoxine HCl | 0.5 mg/L | |
| | Thiamine HCl | 0.1 mg/L | ; a 100-fold (100X) vitamin stock solution was used at 10 mL/L each. |
| | Sucrose | 30 g/L | |
| | Gelrite | 0.25% | |
| | IAA | 0.2 mg/L | |
| | Zeatin | 2 mg/L | |
| | PPT | 5 mg/L | |
| | Carbenicillin | 5 mg/L | |
| | The medium was prepared, and then the pH was adjusted to 5.7 using 1 N NaOH, and then sterilized in an autoclave at 121°C for 15 minutes, and about 30 mL was dispensed into a 90×15 mm petridish. After sterilizing the medium, the vitamin stock solution, BA, NA and AS were added when the temperature of medium was between 55°C and 60°C. | | |

**[Table 8]**

| | | |
|---|---|---|
| Rooting medium | 1/2 MS SALT (including vitamin) | 2.2 g/L |
| | Sucrose | 30 g/L |
| | Plant Agar | 0.6 to 0.7%/L |
| | IAA | 0.2 mg/L |
| | Carbenicillin | 300 mg/L |
| | The medium was prepared, and then the pH was adjusted to 5.7 using 1 N NaOH, and then sterilized in an autoclave at 121°C for 15 minutes, and it was dispensed into a magenta box. After sterilizing the medium, IAA was added when the temperature of medium was between 55°C and 60°C. | |

**[Table 9]**

| Preparation of hormone and vitamin stock solution | | |
|---|---|---|
| | Conc. | Product name or preparing method |
| BA | 1 mg/mL | Caisson (BSL01-100mL) 1 mg/mL solution was purchased and used. |
| NAA | 1 mg/mL | Caisson (NSL01-100mL) 1 mg/mL solution was purchased and used. |
| Zeatin | 2 mg/mL | phytotechlab (Z860) 1 mg/mL solution was purchased and used. |
| IAA(Indole acetic acid) | 1 mg/mL | 50 mL of Duchefa, I0901 reagent was dissolved in 50 mL of D.W, and the mixture was filtered and then dispensed, and stored in a freezer. |
| Acetosyringone (AS) | 100 mM/mL | 0.5887 g of acetosyringone was dissolved in 20 mL of DMSO, and 10 mL of D.W was added to make a total of 30 mL. The prepared solution was dispensed by 1 mL and stored in a freezer (not frozen). |
| Hy gromycin | 10 mg/mL | Biomedic (CAT.No.BM463H) 10 mg/mL solution was purchased and used. |
| Carbenicillin | 300 mg/mL | 1.5 g of Duchefa (C0109) reagent was dissolved in 50 mL of D.W, and the mixture was filtered and then dispensed, and stored in a freezer. |
| Modified Vitamin solution | 100x | It was prepared as a 100-fold stock solution and used when preparing the medium. |
| | | Myo-inositol (MB cell, MB-14715) 10 g/L; nicotinic acid (Showa, 1414-0130) 50 mg/L; pyridoxine HCl (Sigma, P-8666) 50 mg/L; and thiamine HCl (Duchefa, T0614) 10 mg/L were dissolved in 1 L of D.W, and sterilized in an autoclave at 121°C for 15 minutes, and then stored in a refrigerator. |

Specifically, tomato seeds were disinfected with 70% ethanol for 5 seconds and disinfected with 2% bleach for 20 minutes. The disinfected seeds were washed with sterilized water 4 to 5 times, and then placed on sterilized filter paper to remove moisture. The disinfected tomato seeds were transferred to a seed sowing medium TMS (Table 3) and cultured under dark culture conditions at 25°C for 3 days, and then cultured under light culture conditions. After sowing, when the cotyledon grew by 1 cm to 1.5 cm (9 to 12 days), the top of the hypocotyl of the tomato was cut to 0.7 cm to 1.0 cm, and about 18 to 20 (4, 5, 5 and 4, or 5, 5, 5 and 5 each for a total of 18 to 20) were planted on a TPC medium plate (Table 4) and cultured for 1 day under light culture conditions (16L/8D).

Agrobacteria (GV3101 strain, DSMZ, CAT.No.DSM12364) were cultured under dark culture conditions at 28°C for 1 to 2 days in 50 mL of bacterial culture medium (YEP) containing 100 mM AS (acetosyringone) and 50 mg/L rifampicin, 50 mg/L kanamycin (Km) to obtain 0.8 to 1.2 of an OD600 value.

The cultured bacteria were centrifuged at 4°C and 6,000 rpm for 15 minutes to remove the supernatant, and the pellet was resuspended in the same amount of Tomato Co-Culture (TCC) medium (Table 5) and diluted to obtain 0.5 of an OD value.

40 mL of the bacterial culture solution (0.5 of OD600 value) prepared by the above method and the plant body pretreated under light culture conditions were placed in a 50 mL tube (based on sowing of 200 seeds) and cultured for 20 to 30 minutes at room temperature at 25°C. The co-cultured explants were transferred to sterilized filter paper and dried to remove moisture as much as possible, and then about 18 to 20 (4, 5, 5 and 4, or 5, 5, 5 and 5 each for a total of 18 to 20) were planted on a TCC medium plate (Table 5) and cultured for 2 days at 25°C under light culture conditions (16L/8D). After 2 days of co-cultivation, the explants were transferred to a shoot selection medium (TSI, Table 7) and then cultured in a plant incubator at 25°C under light culture conditions (16L/8D). At this time, when Agrobacteria were growing, 300 mg/L carbenicillin was added to a co-culture liquid medium, washed, and then dried on filter paper, and then planted on the medium again.

The callus was differentiated from the cut section between 3 and 4 weeks after planting. When shoots were induced from the differentiated callus and grew more than 1 cm, they were transferred to a rooting medium (Table 9) to induce roots. At this time, the callus was removed as much as possible, and uprooting was induced by transplanting the shoot part. When the root uprooting was fully made, the roots were washed with tap water to remove the Agrobacteria remaining on the roots, transferred to soil, and cultured while adapting to the external environment (humidity change and non-sterile conditions) while maintaining the culture room environment.

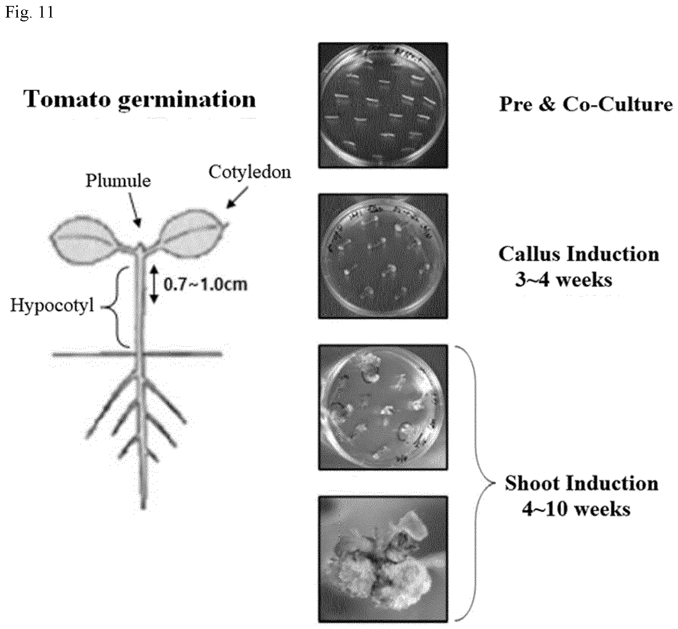

The changes of the objects according to the stage of plant body differentiation in the callus are shown in Fig. 11.

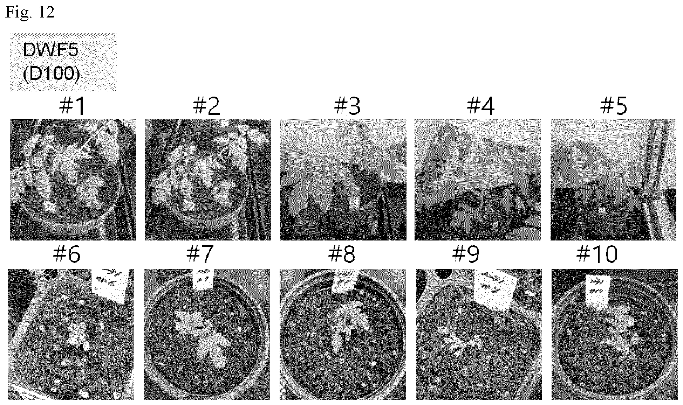

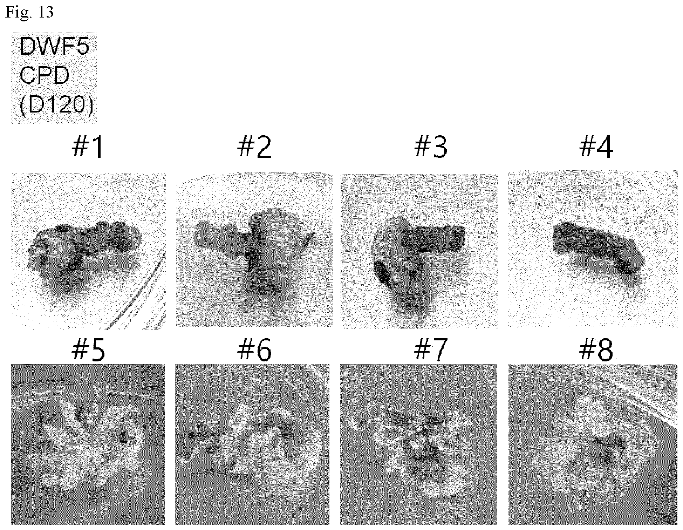

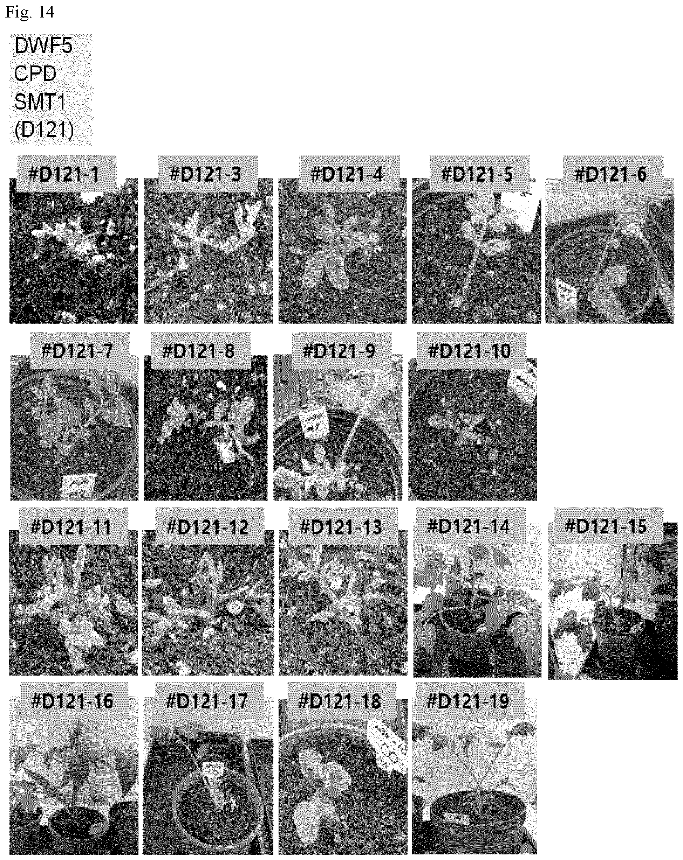

As a result, as shown in Figs. 12 to 14, the tomato shoots transformed with D100 (Fig. 12) or D121 (Fig. 14) grew normally as plant bodies, but the tomato explants transformed with D120 did not grow normally (Fig. 13).

### Example 3. Sequencing of tomato plant body generated by DWF5, CPD and SMT1 gene editing

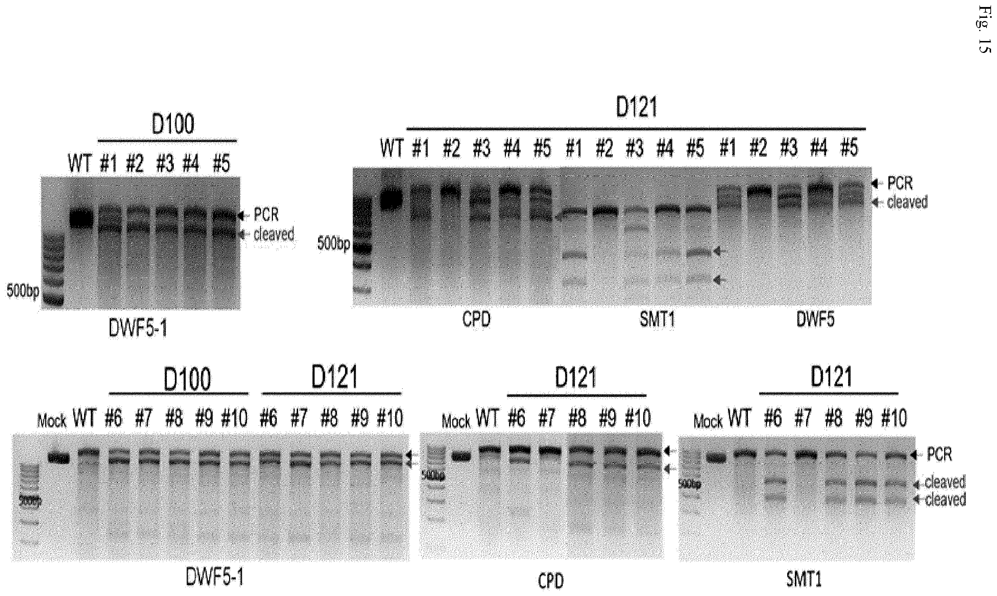

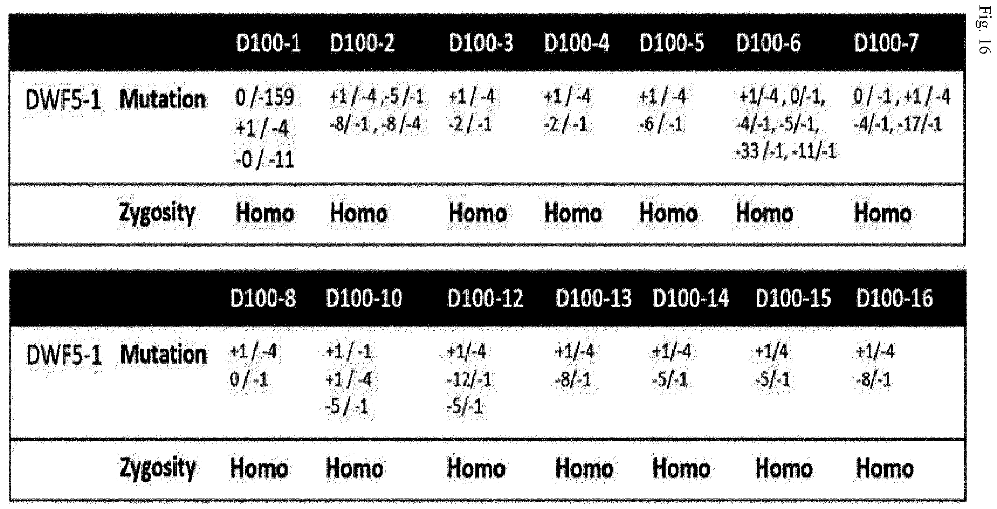

In order to confirm the transformation of the tomato plant body obtained in Example 2, T7E1 assay (Fig. 15) and Sanger sequencing (Fig. 16 and Table 12) were performed on the tomato objects transformed with D100 or D121.

Specifically, in the T7E1 assay, the target region was first amplified through PCR, and then the amplified PCR product was purified using a PCR purification kit. Using the purified PCR product as a DNA template, heteroduplex formation was performed under the conditions of Table 11 and the combinations shown in Table 10. Then, 1 µL of T7 endonuclease I (T7E1) was added and reacted at 37°C for 30 minutes. Finally, it was treated with 1 µL of Proteinase K and reacted at 37°C for 5 minutes to inactivate the activity of T7E1 enzyme. The gene editing efficiency was evaluated to the extent of a fragment concentration in the PCR product.

**[Table 10]**

| | |
|---|---|
| PCR Product (250 ng) | 5 µL |
| 10x NEB Buffer 2 | 2 µL |
| Nuclease-free water | Up to 14 µL |

**[Table 11]**

| Temperature (°C) | Ramp rate |
|---|---|
| 95 | 4.0/sec |
| 95-85 | -2.0/sec |
| 85-25 | -0.1/sec |
| 25-4 | 4.0/sec |

Sanger sequencing was performed as follows. The sgRNA target region was amplified from genomic DNA extracts using Q5 High-Fidelity DNA Polymerase (NewEngland Biolabs) in 20 µL reaction volumn. Thereafter, the PCR product was cloned into a TA vector using an All in one Cloning Kit (Biofact, South Korea), and 15 to 20 cloned clones were individually sequenced for each sample. As a result, 14 tomato homozygotes with DWF5 gene deletion produced by transformation with D100 were identified (Figs. 15 and 16). In addition, one tomato homozygote with DWF5, CPD and SMT1 gene deletion transformed with D121 was identified (Fig. 15 and Table 12). In the case of a D121 transformed plant body, plant hormone imbalance caused by gene deletion resulted in a phenomenon in which fruit was not formed.

**[Table 12]**

| Sample | | D121-4 | D121-5 | D121-6 | D121-7 | D121-8 | D121-9 | D121-10 |
|---|---|---|---|---|---|---|---|---|
| DWF5-1 | Mutation | 0/-158; 2 | -157; 3 | -1; 4 | +1/-4; 6 | -158; 5 | -659; 1 | -4; 1 |
| | | | -5/-5; 1 | | -13/1, 0/-1, | -7, -4/-7; 1 | -8/0, -6/0, | -6; 3 |
| | | WT; 18 | -5/-3; 1 | WT; 5 | -8/-1, -3/-1; | -6; 1 | -4/0; 1 | +1; 1 |
| | | | -5/-0; 4 | | 1 | WT; 5 | WT; 2 | WT; 7 |
| | | | WT; 11 | | | | | |
| | Zygosity | Hetero | Hetero | Hetero | Homo | Hetero | Hetero | Hetero |
| CPD | Mutation | -136 | -136 | -2; 7 | None | -2; 2 | -2; 2 | -2; 14 |
| | | | | | | -3; 1 | -3; 6 | -3; 2 |
| | | | WT; 2 | -5; 4 | | WT; 6 | WT; 2 | -4; 3 |
| | Zygosity | Homo | Hetero | Homo | WT | Hetero | Hetero | Homo |
| SMT1 | Mutation | -4; 1 | -8; 3 | -4; 1 | None | -4; 4 | -1; 5 | -2; 5 |
| | | -5/-4; 1 | -4; 2 | -2; 3 | | -2; 1 | -2; 6 | -4; 5 |
| | | WT; 9 | WT; 7 | WT; 2 | | WT; 2 | -7; 9 | WT; 1 |
| | | | | | | | -91, WT; 1 | |
| | Zygosity | Hetero | Hetero | Hetero | WT | Hetero | Hetero | Hetero |
| Knock out | | CPD | | CPD | DWF5 | | | CPD |

| Sample | | D121-13 | D121-14 | D121-15 | D121-16 | D121-17 | D121-18 | D121-19 |
|---|---|---|---|---|---|---|---|---|
| DWF5-1 | Mutation | -8; 1 | -8; 2 | -4; 4 | -5; 5 | -7; 2 | -4; 5 | -1; 1 |
| | | | | | | | -6; 2 | -4; 3 |
| | | WT; 9 | WT; 8 | +1; 1 | -5/-15; 1 | WT; 5 | -8/-7; 1 | -8; 1 |
| | | | | WT; 3 | -5/-1; 1 | | +1/-6; 1 | WT; 4 |
| | Zygosity | Hetero | Hetero | Hetero | Homo | Hetero | Homo | Hetero |
| CPD | Mutation | -8; 1 | None | None | -136;8 | None | None | None |
| | | -4; 2 | | | | | | |
| | | WT; 7 | | | -6;2 | | | |
| | Zygosity | Hetero | WT | WT | Homo | WT | WT | WT |
| SMT1 | Mutation | -45; 2 | -1; 2 | -4; 7 | -4; 3 | -1; 3 | -1; 2 | -1; 2 |
| | | | -2; 6 | | | -2; 6 | -2; 5 | -2; 6 |
| | | WT; 4 | -3; 1 | -2; 3 | -8; 5 | -4; 1 | -4; 2 | -4; 2 |
| | Zygosity | Hetero | Homo | Homo | Homo | Homo | Homo | Homo |
| Knock out | | | SMT1 | SMT1 | DWF5 | SMT1 | DWF5 | SMT1 |
| | | | | | CPD | | SMT1 | |
| | | | | | SMT1 | | | |

### Example 4. Obtaining seeds and gene phenotype analysis of tomatoes produced by DWF5 gene editing

The seeds (DWF5 homozygote) obtained from 7 tomatoes of tomatoes (T0 generation) with DWF5 gene deletion through genetic scissors were sown, and next generation sequencing (NGS) was performed to determine whether the tomatoes with DWF5 gene deletion of the next generation (T1) were obtained.

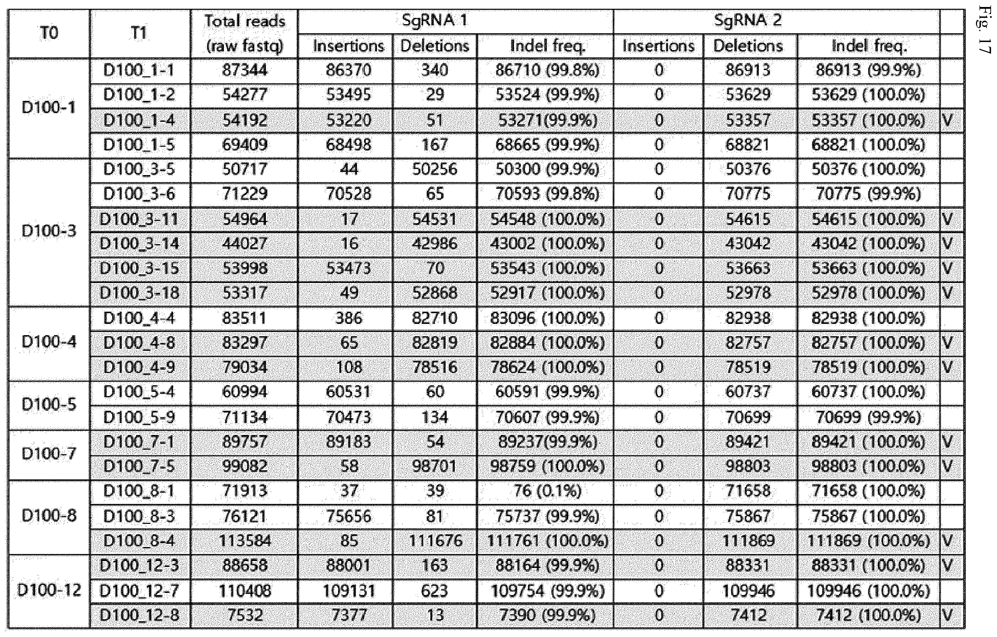

As a result, it was confirmed that 12 of the 23 seeds (T1) obtained from 7 T0 objects were homozygous (Fig. 17). In addition, among the above 12 seeds, 6 seeds whose gene editing efficiency (indel efficiency) was 100% were selected, and the occurrence of seeds (T2) and the amount of seeds obtained were confirmed. As a result, seeds were produced in 5 out of 6 objects (Fig. 18 and Table 13). In addition, no significant differences were observed in the phenotypes of fruits, leaves, and stems of the tomatoes with DWF5 gene deletion compared to the wild type tomato. At this time, the wild type tomato (wild type, WT) is a control group.

**[Table 13]**

| Label | Total reads | sgRNA 1 | | | sgRNA 2 | | | T1 Zygote | Amount of T2 seeds (g) | |
|---|---|---|---|---|---|---|---|---|---|---|
| | | Insertions | Deletions | Indel freq. (%) | Insertions | Deletions | Indel freq. (%) | | | |
| D100-1-4 | 54192 | 53220 | 51 | 100% | 0 | 52983 | 100% | Mono-allel ic | 0.37 | 1.06 |
| D100-7-1 | 89757 | 89183 | 54 | 100% | 0 | 88705 | 100% | Mono-allel ic | 0.35 | |
| D100-12 -8 | 7532 | 7377 | 13 | 100% | 0 | 7267 | 100% | Mono-allel ic | 0.34 | |
| D100-3-11 | 54964 | 0 | 54465 | 100% | 0 | 53656 | 100% | Mono-allel ic | 0.13 | 0.45 |
| D100-3-14 | 44027 | 0 | 42936 | 100% | 0 | 42304 | 100% | Mono-allel ic | 0.32 | |
| D100-4-8 | 83297 | 0 | 82686 | 100% | 0 | 81960 | 100% | Mono-allel ic | - | |

### Example 5. Phenotype analysis of T2 generation

### Example 5.1. Analysis of vitamin D3 precursor

The seeds (T2) obtained in Example 4 were sown, and the content of provitamin D3 contained in T2 generation tomatoes was measured by LC/MS. At this time, the wild type tomato (WT), the T2 generation (4 objects) of #3-14 line (D100-3-14) and the T2 generation (11 objects) of #7-1 line (D100-7-1) were used as a sample. 7-dehydrocholesterol, a precursor of animal provitamin D3 (provitamin D3, sigma, Cat30800, Lot.BCBS6021) was used as a standard reagent. In addition, as a control group, the wild type (WT) tomato was used as a sample.

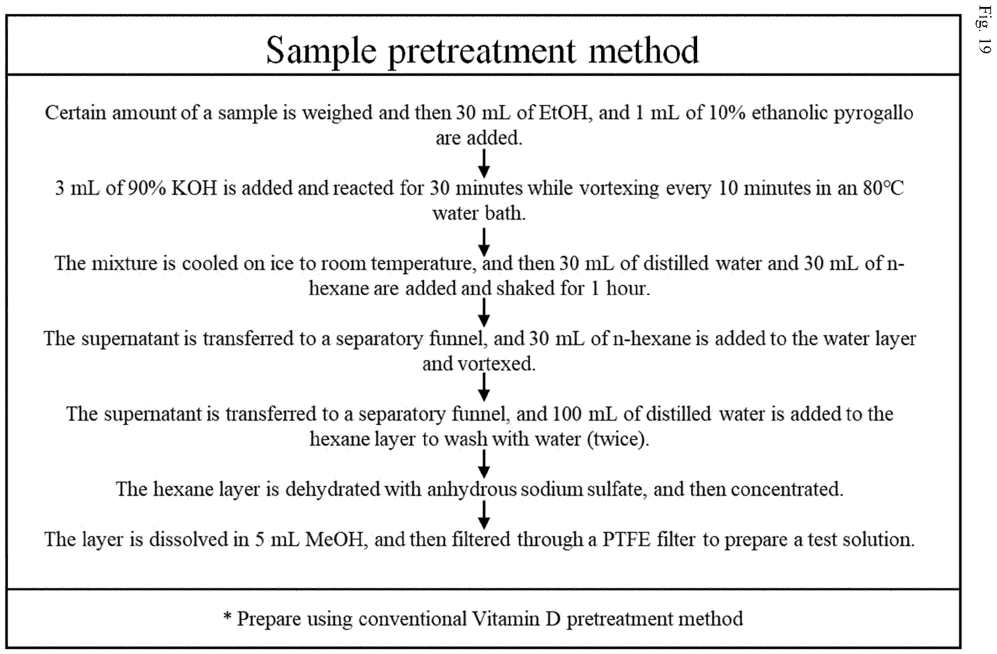

Specifically, certain amount of each sample was weighed, and 30 mL of ethanol and 1 mL of 10% ethanolic pyrogallo were added. Thereafter, 3 mL of 90% potassium hydroxide (KOH) was added and reacted for 30 minutes while vortexing every 10 minutes in an 80°C of water bath. After the reaction was completed, the mixture was cooled to room temperature, and then 30 mL of distilled water and 30 mL of n-hexane were added, and mixed by shaking for 1 hour. Thereafter, the supernatant was transferred to a separatory funnel, and 30 mL of n-hexane was added to the water layer and mixed by vortexing. The supernatant was again transferred to a separatory funnel, and 100 mL of distilled water was added to the hexane layer to wash with water. The washing process with water was repeated twice. Thereafter, the hexane layer was dehydrated with anhydrous sodium sulfate, and then concentrated, dissolved in 5 mL methanol (MeOH), and filtered through a polytetrafluoroethylene (PTFE) filter to prepare a test solution (Fig. 19).

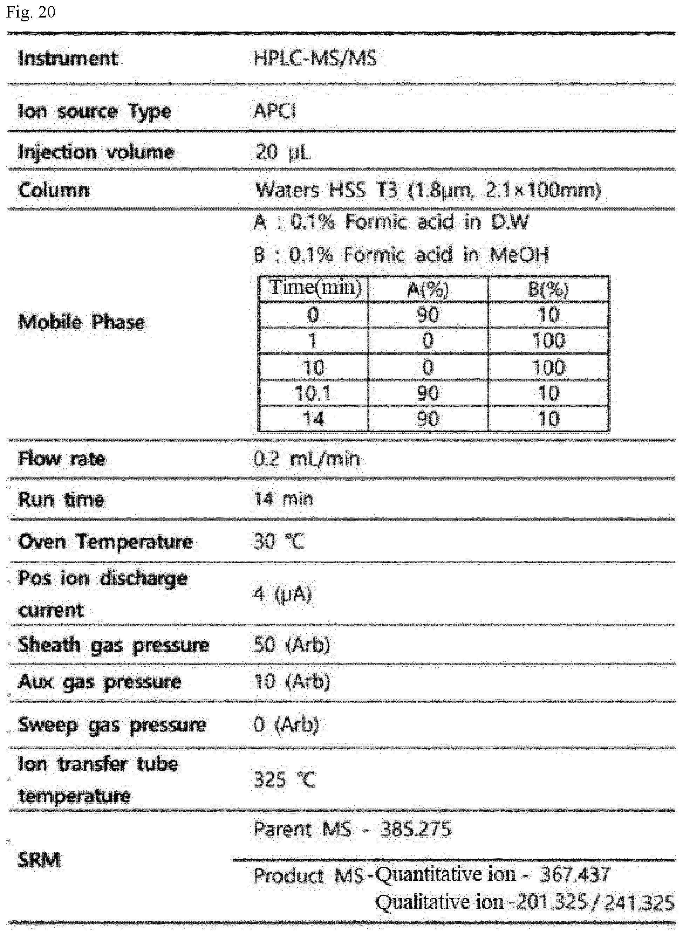

7-dehydrocholesterol contained in the test solution prepared by the above process was measured through LC/MS under the conditions of Fig. 20.

As a result, 7-dehydrocholesterol was not detected in the fruit parts of WT tomatoes, whereas 7-dehydrocholesterol was detected in 4 objects in #3-14 line of the T2 generation and 11 objects in #7-1 line (a total of 15 objects) (Table 14, Fig. 21 and Fig. 22).

**[Table 14]**

| No | Sample Name | Sample Status | Final Volume (L) | Standard Form Purity (%) | Results (mg/100 g) |
|---|---|---|---|---|---|
| 1 | WT tomato | mature fruit | 0.005 | 95.3 | Not detected |
| 2 | WT tomato | mature fruit | 0.005 | 95.3 | Not detected |
| 3 | #3-14-2 tomato | mature fruit | 0.005 | 95.3 | 1.03 |
| 4 | #3-14-13 tomato | mature fruit | 0.005 | 95.3 | 0.60 |
| 5 | #3-14-20 tomato | mature fruit | 0.005 | 95.3 | 0.19 |
| 6 | #3-14-22 tomato | mature fruit | 0.005 | 95.3 | 0.26 |
| 7 | #7-1-2 tomato | mature fruit | 0.005 | 95.3 | 0.23 |
| 8 | #7-1-7 tomato | mature fruit | 0.005 | 95.3 | 0.12 |
| 9 | #7-1-12 tomato | including immature fruit | 0.005 | 95.3 | 0.27 |
| 10 | #7-1-14 tomato | leaf stem | 0.005 | 95.3 | 0.02 |
| 11 | #7-1-17 tomato | mature fruit | 0.005 | 95.3 | 0.14 |
| 12 | #7-1-18 tomato | leaf stem | 0.005 | 95.3 | 0.03 |
| 13 | #7-1-20 tomato | immature fruit | 0.005 | 95.3 | 0.36 |
| 14 | #7-1-21 tomato | leaf stem | 0.005 | 95.3 | Not detected |
| 15 | #7-1-22 tomato | leaf stem | 0.005 | 95.3 | Not detected |
| 16 | #7-1-24 tomato | immature fruit | 0.005 | 95.3 | 0.71 |
| 17 | #7-1-27 tomato | leaf stem | 0.005 | 95.3 | 0.03 |

### Example 5.2. Analysis of vitamin D3 precursor in tomato roots

The seeds (T2) obtained in Example 4 were disinfected with 70% ethanol for 5 seconds and disinfected with 2% bleach for 20 minutes. The disinfected seeds were washed with sterilized water 4 to 5 times, and then placed on sterilized filter paper to remove moisture. The seeds were sown in an MS medium (Table 15) and cultured at 25°C under 16-hour light/8-hour dark cycle conditions. After 2 to 3 weeks, when roots were formed, the roots were taken out from the plate, and then the roots (hairy roots) were collected and analyzed for 7-DHC content by GC-MS method.

**[Table 15]**

| | |
|---|---|
| Composition of medium | 1 L |
| MS powder | 4.4 g |
| Sucrose | 10g |
| pH 5.8 | |
| Plant agar | 7 g |

GC-MS metabolic analysis was performed by mixing 10 mg of root with 3 mL of 0.1% ascorbic acid-ethanol and 0.05 mL of 5α-cholestane to obtain the root extract. The extract was mixed with 80% potassium hydroxide to proceed to saponification, and then mixed with hexane to separate lipophilic substances. The separated lipophilic substances were derivatized using pyridine and N-methyl-N-trimethylsilyl trifluoroacetamide, and then GC-MS (gas chromatography-quadrupole mass spectrometry) analysis was performed. For quantitative and qualitative analysis, calibration curves calculated from standard materials were used.

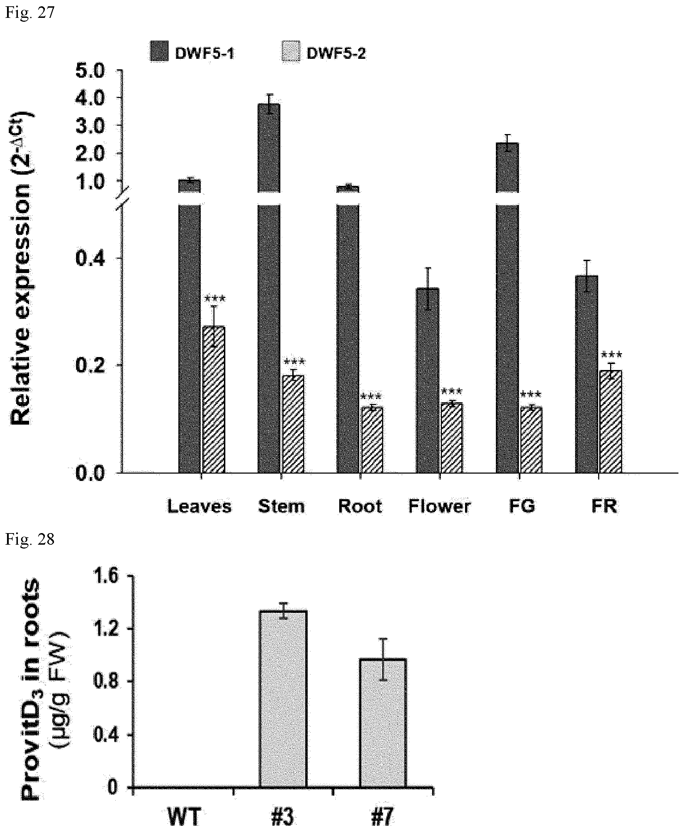

As a result, 7-dehydrocholesterol was not detected in the roots of WT tomatoes, whereas 7-dehydrocholesterol was detected at concentrations of 1.3 µg/g and 1.0 µg/g in #3 line (3-14-29) and #7 line (7-1-15) of the T3 generation, respectively (Fig. 28).

### Example 5.3. Selection of antibiotic-free object in T2 generation

In order to select objects without antibiotic resistance among #3-14 line (D100-3-14) and #7-1 line (D100-7-1) of the T2 generation obtained in Example 4, the expression of the antibiotic resistance gene in the mRNA of the objects was confirmed through RT-PCR.

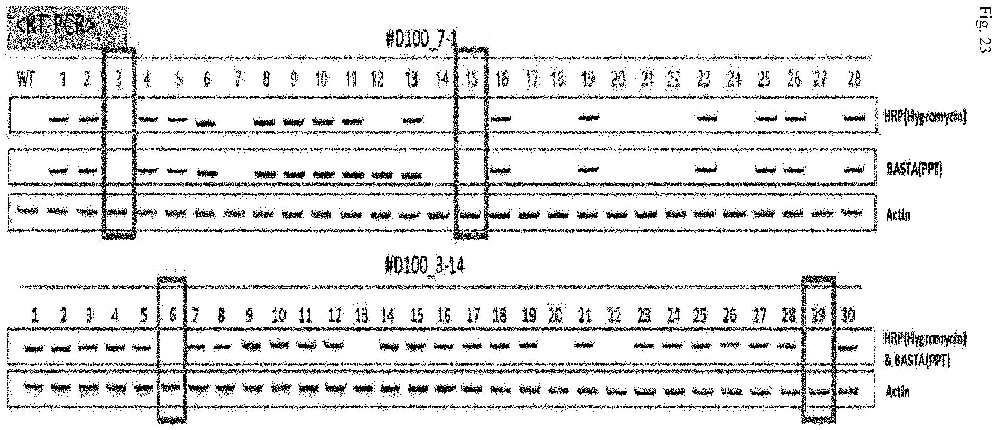

As a result, it was confirmed that 5 objects in #3-14 line (D100-3-14) and 11 objects in #7-1 line (D100-7-1) were objects without antibiotic resistance (Fig. 23).

Among the objects selected as objects without antibiotic resistance, #3-14-29 and #7-1-15 objects were checked for DWF5 gene editing.

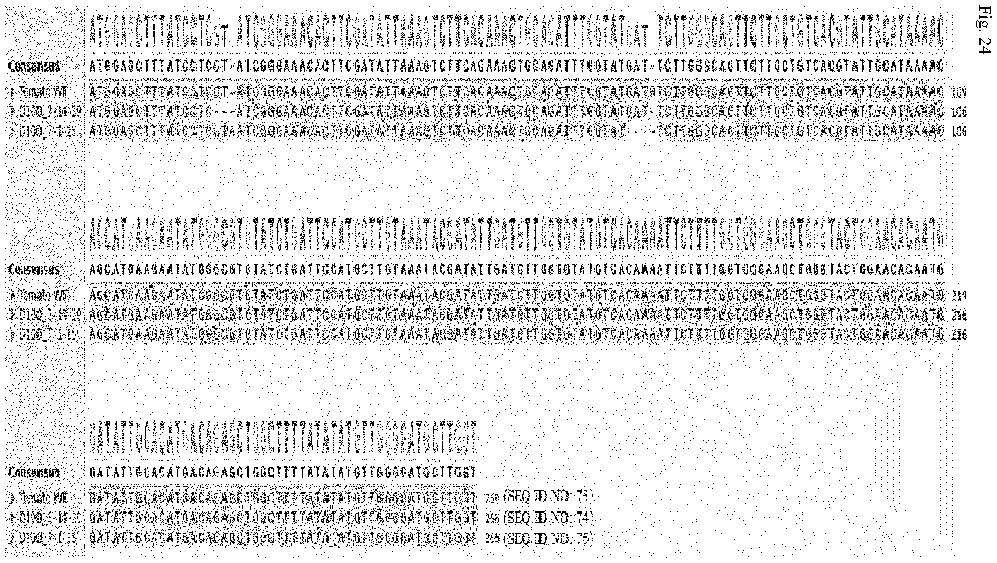

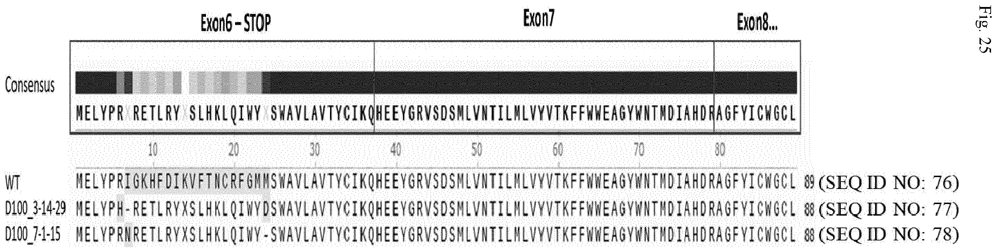

As a result, as shown in Figs. 24 and 25, it was confirmed that the nucleotide sequence of the object was different from that of the wild type (WT) tomato (Fig. 24), and the amino acid sequence was also different from that of the wild type (WT) tomato because the sequence corresponding to exon 6 was deleted (Fig. 25).

### Example 5.4. Confirmation of phenotype and seed generation of T2 generation

The #3-14 line (D100-3-14) and #7-1 line (D100-7-1) of the T2 generation obtained in Example 4 were checked for phenotype and seed production. Specifically, when only the DWF5 gene (DWF5-1, SEQ ID NO: 63), which is mainly expressed in fruits, was deleted, no significant difference was observed in the phenotypes of fruits, leaves, and stems compared to the wild type tomato (Fig. 27). Therefore, in the present invention, by deleting only the DWFS-1 (SEQ ID NO: 63) gene, it was possible to produce tomatoes capable of supplementing the problem of seed generation while inducing enrichment of provitamin D3.

## Claims

1. A transformed tomato containing a high concentration of 7-dehydrocholesterol of Formula 1 below.

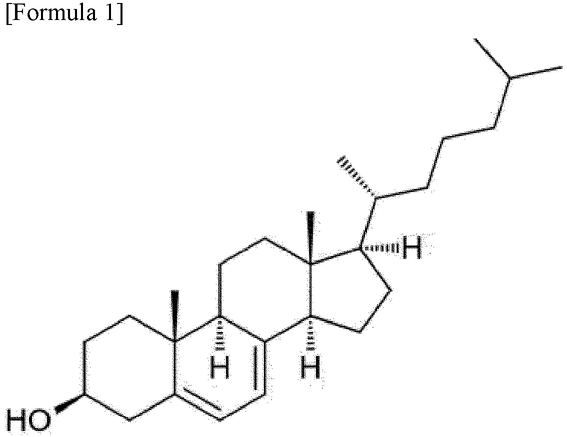

2. The transformed tomato according to claim 1, wherein 7-dehydrocholesterol is contained in a high concentration in a fruit and a root of the tomato.

3. The transformed tomato according to claim 1, wherein the concentration of 7-dehydrocholesterol is 0.01 mg to 1.5 mg per 100 g of weight.

4. The transformed tomato according to claim 1, wherein the transformed tomato is genetically engineered to reduce the expression or activity of the DWFS-1 gene or DWF5-1 protein compared to the wild type tomato.

5. The transformed tomato according to claim 4, wherein the DWFS-1 gene comprises the nucleic acid sequence of SEQ ID NO: 63.

6. The transformed tomato according to claim 4, wherein the genetic engineering is induced by modification in the nucleic acid sequence of the DWFS-1 gene.

7. The transformed tomato according to claim 6, wherein the modification in the nucleic acid sequence comprises a sequence different from that of the wild type tomato by substitution, deletion or insertion.

8. The transformed tomato according to claim 4, wherein the tomato is further genetically engineered to reduce the expression or activity of the CPD gene or CPD protein.

9. The transformed tomato according to claim 8, wherein the CPD comprises the nucleic acid sequence of SEQ ID NO: 5.

10. The transformed tomato according to claim 8, wherein the genetic engineering is induced by modification in the nucleic acid sequence of the CPD gene.

11. The transformed tomato according to claim 8, wherein the tomato is further genetically engineered to reduce the expression or activity of the SMT1 gene or SMT1 protein.

12. The transformed tomato according to claim 11, wherein the SMT1 comprises the nucleic acid sequence of SEQ ID NO: 7.

13. The transformed tomato according to claim 11, wherein the genetic engineering is induced by modification in the nucleic acid sequence of the SMT1 gene.

14. The transformed tomato according to claim 4, wherein the transformed tomato is a homozygote.

15. The transformed tomato according to claim 4, wherein the transformed tomato forms seeds.

16. A vector comprising at least one sgRNA (single guide RNA) that complementarily binds to a nucleotide sequence of the DWFS-1 gene and a nucleotide sequence encoding a CRISPR (clustered regularly interspaced palindromic repeats)-associated protein.

17. The vector according to claim 16, wherein the sgRNA comprises any one nucleotide sequence selected from the group consisting of SEQ ID NO: 20, SEQ ID NO: 21 and a combination thereof.

18. The vector according to claim 16, wherein the vector further comprises one or more than one sgRNA that complementarily binds to a nucleotide sequence of the CPD gene.

19. The vector according to claim 18, wherein the sgRNA comprises any one nucleotide sequence selected from the group consisting of SEQ ID NO: 28, SEQ ID NO: 29 and a combination thereof.

20. The vector according to claim 16, wherein the vector further comprises one or more than one sgRNA that complementarily binds to a nucleotide sequence of the SMT1 gene.

21. The vector according to claim 20, wherein the sgRNA comprises any one nucleotide sequence selected from the group consisting of SEQ ID NO: 32, SEQ ID NO: 33 and a combination thereof.

22. The vector according to claim 16, wherein the CRISPR-associated protein is any one selected from the group consisting of Cas9, Cpf1, c2c1, C2c2, Cas13, c2c3, Cas1, Cas1B, Cas2, Cas3, Cas4, Cas5, CasSe (CasD), Cas6, Cas6e, Cas6f, Cas7, Cas8a, Cas8a1, Cas8a2, Cas8b, Cas8c, Csn1, Csx12, Cas10, Cas10d, Cas10, Cas10d, CasF, CasG, CasH, Csyl, Csy2, Csy3, Cse1 (CasA), Cse2 (CasB), Cse3 (CasE), Cse4 (CasC), Csc1, Csc2, Csa5, Csn2, Csm2, Csm3, Csm4, Csm5, Csm6, Cmrl, Cmr3, Cmr4, Cmr5, Cmr6, Csb1, Csb2, Csb3, Csx17, Csx14, Csx10, Csx16, CsaX, Csx3, Csxl, Csx15, Csf1, Csf2, Csf3, Csf4 and Cul966.

23. The vector according to claim 22, wherein the CRISPR-associated protein is Cas9.

24. A method for producing a transformed tomato containing a high concentration of 7-dehydrocholesterol, comprising introducing the vector according to any one of claims 16 to 23 into a tomato using Agrobacterium.
